# EUROPEAN PATENT APPLICATION

(11) **EP 4 368 620 A1**
(43) Date of publication of application: **15.05.2024**
(21) Application number: 22836931.0
(22) Date of filing: 05.07.2022
(51) Int. Cl.: C07D 413/02, C07D 413/06, C07D 487/04, A61P 35/00, A61P 35/04, A61K 31/506, A61K 31/41, A61K 31/343

(54) **COMPOUND SERVING AS KAT6 INHIBITOR**

(30) Priority: 05.07.2021 CN 202110758732; 14.01.2022 CN 202210043389; 28.02.2022 CN 202210190640
(71) Applicant: Hangzhou Innogate Pharma Co., Ltd., Hangzhou, Zhejiang 311121 (CN)
(72) Inventor: ZHANG, Hancheng, Hangzhou, Zhejiang 311121 (CN); JIA, Wei, Hangzhou, Zhejiang 311121 (CN); CAI, Congcong, Hangzhou, Zhejiang 311121 (CN)
(74) Representative: Lavoix
(86) International application number: PCT/CN2022/104005
(87) International publication number: WO 2023/280182

(57) **Abstract**

Provided in the present invention is a compound serving as a KAT6 inhibitor. Specifically, provided in the present invention is a compound having a structure as represented by the following formula (I) and formula (XIV), or an optical isomer, a pharmaceutically acceptable salt, a prodrug, a deuterated derivative, a hydrate or a solvate thereof. The compound can be used for treating or preventing diseases or conditions associated with the activity or expression level of KAT6.

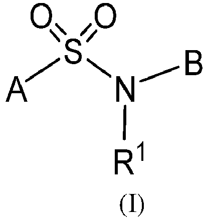

## Description

### Technical Field

The present invention relates to the field of medicinal chemistry; specifically, the present invention relates to a novel class of derivatives containing a tricyclic heteroaryl group, its synthesis and its application as a KAT6 inhibitor in the preparation of drugs for use in the treatment of a wide range of diseases related to tumors and the like.

### Background of Invention

Histone acetyltransferases use acetyl-CoA to transfer acetyl groups to lysine residues of histone or other substrate proteins. Acetylation is a reversible post-translational modification that plays an important role in eukaryotic gene expression, cell cycle, and signaling pathway transduction. According to homology, histone acetyltransferases are divided into four families, namely GNAT (Gcn5-related acetyltransferase) family, p300/CBP family, SRC/p160 family, and MYST (MOZ, Ybf2/Sas3, Sas2 and Tip60) family. The acetyltransferase activity of MYST family is affected by the MYST domain (catalytic domain). The MYST domain contains an acetyl-CoA-binding motif and a rare C2HC-type zinc finger structure, where the acetyl-CoA-binding motif is structurally conserved with other histone acetyltransferases. The MYST domain is the defining feature of MYST family proteins.

KAT6A (Lysine Acetyltransferase 6A, also known as MOZ) and KAT6B (Lysine Acetyltransferase 6B, also known as MORF) are acetyltransferases belonging to the MYST family. KAT6A is chromosomally translocated in acute myeloid leukemia and has amplification mutations in lung cancer, breast cancer, ovarian cancer, endometrial cancer, bladder cancer, and esophageal cancer. Similarly, KAT6B is also chromosomally translocated in a variety of cancer types. MOZ- and MORF-linked fusion proteins identified in acute myeloid leukemia include MOZ-CBP, MOZ-p300, MOZ-TIF2, MOZ-NcoA3, MOZ-LEUTX, and MORF-CBP. MOZ-TIF2 has transforming activity in cultured cells and can induce acute myeloid leukemia in mice. In tumor cells with KAT6A and KAT6B amplification, the expression of KAT6A and KAT6B is closely related to the gene copy number, indicating that there is selective pressure to maintain their activity during tumorigenesis. In addition, in cell proliferation tests, tumor cells with high expression of KAT6A and KAT6B are usually more dependent on the activity of KAT6A and KAT6B.

KAT6A and KAT6B generally extensively acetylate the lysine 23 site of histone H3 (H3K23), and KAT6A can also modify the acetylation of the H3K9 site of its regulatory gene. KAT6A interacts with some transcription factors such as p53 and RUNX1 to acetylate histones and regulate the expression of downstream genes. KAT6A binds to the proximal promoter region of the estrogen receptor ERα (Estrogen Receptor cc) to activate the expression of ERα genes. In ER+, KAT6A amplification or high expression breast cancer cells, inhibition of KAT6A acetyltransferase activity or knockdown of KAT6A can significantly inhibit the proliferation of breast cancer cells. In addition, KAT6A acetyltransferase activity is essential for promoting the expression of MEIS 1 and HOXa9 genes, which are often overexpressed in some lymphoma and leukemia cells. In MYC-induced lymphoma mouse model, the deletion of a KAT6A allele can significantly prolong the median survival of mice. In mice, KAT6B allele mutations lead to a significant reduction in the division and differentiation of cortical progenitors, which seriously affects the development of the cerebral cortex. KAT6B also plays an important role in maintaining the number of adult neural stem cells. KAT6B is also mutated in some rare types of leukemia.

Based on the above background, we developed a novel series of inhibitors of KAT6.

### Description of the invention

It is an object of the present invention to provide a novel class of KAT6 inhibitors.

In the first aspect of the present invention, a compound of the following formula (I), or the optical isomers, pharmaceutically acceptable salts, prodrugs, deuterated derivatives, hydrates, or solvates thereof is provided: 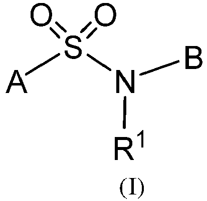 wherein in the formula (I):
A is selected from Formula (Ia), Formula (Ib), Formula (Ic) and Formula (Id): 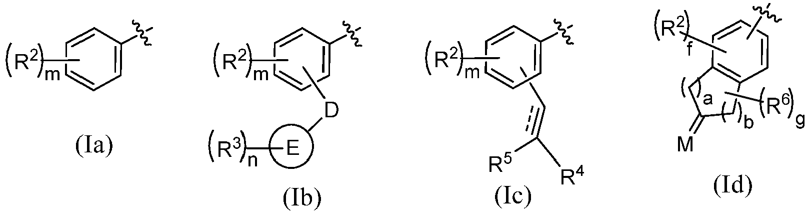
wherein, " 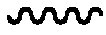 " refers to the site of formula (Ia) which attach to the remaining fragment of the compound of formula (I);
" 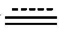 " represents a double or triple bond; and R⁵ is absent when it is a triple bond;
B is selected from Formula (Ie), Formula (If) and Formula (Ig): 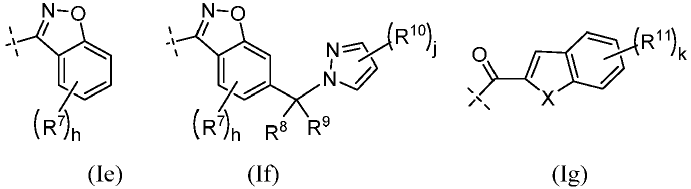
wherein, the "---" refers to the site of the fragment B which attach to the remaining fragment of the compound of formula (XIa);
R¹ is selected from the group consisting of hydrogen and C₁₋₄ alkyl;
each R² is independently selected from the group consisting of hydrogen, halogen, C₁₋₄ alkyl, C₁₋₄ haloalkyl, C₁₋₄ alkoxy-C₁₋₄ alkyl, C₁₋₄ haloalkoxy-C₁₋₄ alkyl, C₂₋₄ alkenyl, C₂₋₄ haloalkenyl, C₂₋₄ alkynyl, C₃₋₆ cycloalkyl, 3-to 8-membered heterocyclyl, aryl, heteroaryl, CN, OR^{a}, SR^{a}, and NR^{a}R^{a}; wherein, each R^{a} is independently selected from hydrogen, C₁₋₄ alkyl, C₁₋₄ haloalkyl, C₃₋₆ cycloalkyl, and 3-to 8-membered heterocyclyl;
D is selected from chemical bond, -O-, -S-, and-NR^{e}-; wherein R^{e} is selected from hydrogen, C₁₋₄ alkyl, and C₃₋₆ cycloalkyl; E is selected from C₃₋₈ cycloalkyl, 3-to 10-membered heterocyclyl, C₃₋₈ cycloalkyl-C₁₋₄ alkyl, and 3-to 10-membered heterocyclyl-C₁₋₄ alkyl;
each R³ is independently selected from hydrogen, halogen, C₁₋₄ alkyl, C₁₋₄ haloalkyl, CN, OR^{b}, SR^{b}, NR^{b}R^{b}, -C(O)R^{g}, -S(O)₂R^{g}, C₃₋₆ cycloalkyl, and 3-to 8-membered heterocyclyl; or two R³ which attach to a same carbon atom of the cycloalkyl or heterocyclyl ring form C=T together with the carbon atom; wherein, T is selected from O or CR¹²R¹³; wherein, R¹² and R¹³ are each independently selected from hydrogen, fluorine, or C₁₋₄ alkyl; the alkyl on the R¹² or R¹³ is optionally substituted with one or more groups selected from the group consisting: halogen, C₁₋₄ haloalkyl, CN, OR^{b}, SR^{b}, NR^{b}R^{b}, C₃₋₆ cycloalkyl, 3-to 8-membered heterocyclyl, aryl, and heteroaryl; each R^{b} is independently selected from hydrogen, C₁₋₄ alkyl, and C₁₋₄ haloalkyl; R^{g} is selected from hydrogen, C₁₋₄ alkyl, C₂₋₄ alkenyl, C₂₋₄ alkynyl, C₃₋₈ cycloalkyl, 3-to 8-membered heterocyclyl, aryl, and heteroaryl;
R⁴ and R⁵ are each independently selected from the group consisting of hydrogen, C₁₋₄ alkyl, C₃₋₈ cycloalkyl, 3-to 9-membered heterocyclyl, aryl, and heteroaryl; wherein the alkyl, cycloalkyl, heterocyclyl, aryl, or heteroaryl is optionally substituted with one or more groups selected from the group consisting of halogen, CN, OR^{a}, SR^{a}, NR^{a}R^{a}, NO₂, -C(O)R^{m}, -C(O)OR^{a}, -S(O)₂R^{m}, -C(O)NR^{a}R^{a}, -OC(O)R^{m}, -NR^{a}C(O)R^{m}, -NR^{a}S(O)₂R^{m}, C₁₋₄ alkyl, C₂₋₄ alkenyl, C₂₋₄ alkynyl, C₃₋₆ cycloalkyl, 3-to 8- membered heterocyclyl, aryl, and heteroaryl; or the cycloalkyl or heterocyclic group is substituted by =T; wherein, the definition of each R^{a} is as described as above; R^{m} is selected from C₁₋₄ alkyl, C₂₋₄ alkenyl, C₂₋₄ alkynyl, and C₃₋₆ cycloalkyl; the definition of T is described as above;
each R⁶ is independently selected from hydrogen, halogen, C₁₋₄ alkyl, C₁₋₄ haloalkyl, CN, OR^{b}, SR^{b}, and NR^{b}R^{b}; wherein each R^{b} is as defined above;
M is selected from O or CR^{h}Rⁱ; wherein R^{h} and Rⁱ are each independently selected from hydrogen, fluoro, and C₁₋₄ alkyl; wherein, said alkyl is optionally substituted by one or more groups selected from halogen, C₁₋₄ haloalkyl, CN, OR^{b}, SR^{b}, NR^{b}R^{b}, C₃₋₆ cycloalkyl, 3-to 8-membered heterocyclyl, aryl, and heteroaryl; wherein each R^{b} are defined as above;
each R⁷ is independently selected from hydrogen, halogen, C₁₋₄ alkyl, C₁₋₄ haloalkyl, C₂₋₄ alkenyl, C₂₋₄ alkynyl, CN, OR^{a}, SR^{a}, NR^{a}R^{a}, C₃₋₆ cycloalkyl, 3-to 8-membered heterocyclyl, aryl, heteroaryl, and heteroaryl C₁₋₄ alkyl; wherein, each R^{a} is defined as above;
R⁸ and R⁹ are each independently selected from the group consisting of hydrogen, fluoro, C₁₋₄ alkyl, C₁₋₄ alkoxy, hydroxy, and C₃₋₆ cycloalkyl; or R⁸ and R⁹ together with the carbon atom to which they are attached form a 3-to 6-membered ring structure which optionally contains 0, 1 or 2 heteroatoms selected from N, O and S;
each R¹⁰ is independently selected from hydrogen, halogen, C₁₋₄ alkyl, C₁₋₄ haloalkyl, C₂₋₄ alkenyl, C₂₋₄ alkynyl, C₃₋₆ cycloalkyl, 3-to 8-membered heterocyclyl, CN, OR^{a}, SR^{a}, and NR^{a}R^{a}; wherein each R^{a} is as defined above;
X is selected from O, S and NRⁿ; wherein Rⁿ is selected from hydrogen and C₁₋₄ alkyl;
each R¹¹ is independently selected from hydrogen, halogen, C₁₋₄ alkyl, C₁₋₄ haloalkyl, C₂₋₄ alkenyl, C₂₋₄ alkynyl, C₃₋₆ cycloalkyl, 3-to 8-membered heterocyclyl, CN, OR^{a}, SR^{a}, NR^{c}R^{c}, and -NR^{d}C(O)R^{g}; wherein, each R^{a} is defined as above; each R^{c} is independently selected from hydrogen, C₁₋₄ alkyl, C₁₋₄ haloalkyl, C₁₋₄ alkoxy-C₁₋₄ alkyl, C₃₋₆ cycloalkyl, 3-to 8-membered heterocyclyl, aryl, and heteroaryl; R^{d} is selected from hydrogen and C₁₋₄ alkyl; R_{g} is defined as above;
m is selected from 0, 1, 2, 3 and 4;
n is selected from 0, 1, 2, 3 and 4;
f is selected from 0, 1, 2 and 3;
g is selected from 0, 1, 2, 3 and 4;
a and b are each independenly selected from 0, 1, 2, 3, 4 and 5; with the proviso that a and b are not 0 simultaneously;
h is selected from 0, 1, 2 and 3;
j is selected from 0, 1, 2 and 3;
k is selected from 0, 1, 2, 3, 4 and 5;
wherein, each of the above-mentioned alkyl, alkenyl, alkynyl, cycloalkyl, heterocyclyl, aryl and heteroaryl is optionally and each independently substituted by 1-3 substituents each independently selected from the group consisting of halogen, C₁₋₄ alkyl, C₁₋₄ haloalkyl, C₂₋₄ alkenyl, C₂₋₄ alkynyl, C₃₋₈ cycloalkyl, 3-to 8-membered heterocyclyl, aryl, heteroaryl, CN, NO₂, OR^{b}, SR^{b}, NR^{d}R^{d}, -C(O)R^{g}, -C(O)OR^{b}, -C(O)NR^{d}R^{d}, -NR^{d}C(O)R^{g}, -NR^{d}S(O)₂R^{g}, and -S(O)₂R^{g}, provided that the chemical structure formed is stable and meaningful; wherein R^{b}, R^{d} and R^{g} are as defined above;
unless otherwise specified, the aryl is aromatic groups having 6 to 12 carbon atoms; the heteroaryl is 5- to 15-membered (preferably 5- to 12-membered) heteroaromatic groups; and the cyclic structure is saturated or unsaturated cyclic groups with or without heteroatoms.

In another preferred embodiment, the compound (I) is of formula (II): 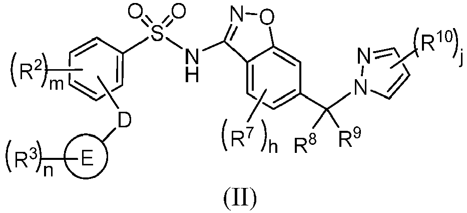 R², R³, R⁷, R⁸, R⁹, R¹⁰, D, E, m, n, h and j are as defined above.

In another preferred embodiment, said E is selected from C₃₋₆ cycloalkyl, 3-to 6-membered heterocyclyl, C₃₋₆ cycloalkyl-C₁₋₄ alkyl, and 3-to 6-membered heterocyclyl-C₁₋₄ alkyl; each R³ is independently selected from hydrogen, halogen, C₁₋₄ alkyl, C₁₋₄ haloalkyl, CN, OR^{b},-C(O)R^{g}; or two R³ attaching to the same carbon atom of the cycloalkyl or heterocyclyl ring together with the carbon atom form C=T; wherein, T is selected from O and CR¹²R¹³; wherein, R¹² and R¹³ are each independently selected from hydrogen, fluorine, and C₁₋₄ alkyl; the alkyl on said R¹² or R¹³ is optionally substituted by one or more groups selected from the group consisting of halogen, C₁₋₄ haloalkyl, OR^{b}, and NR^{b}R^{b}; each R^{b} is independently selected from hydrogen, C₁₋₄ alkyl, or C₁₋₄ haloalkyl; R^{g} is selected from hydrogen, C₁₋₄ alkyl, and C₃₋₈ cycloalkyl.

In another preferred embodiment, the compound (I) is of formula (III): 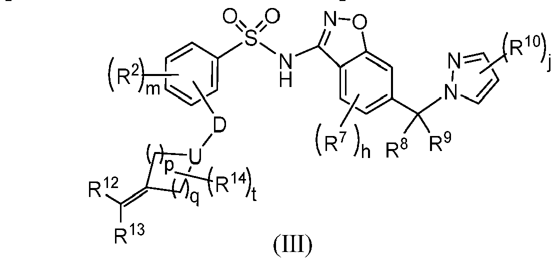
U is selected from N and CR^{k}; wherein R^{k} is selected from hydrogen, halogen and C₁₋₄ alkyl;
each R¹⁴ is independently selected from hydrogen, halogen, C₁₋₄ alkyl, C₁₋₄ haloalkyl, CN, OR^{b}, SR^{b}, and NR^{b}R^{b};
p and q are each independently selected from 0, 1, 2, 3, 4, 5 and 6; with the proviso that p and q are not simultaneously 0;
t is selected from 0, 1, 2, 3 and 4;
R², R⁷, R⁸, R⁹, R¹⁰, R¹², R¹³, R^{b}, D, m, h, and j are as defined above.

In another preferred embodiment, the compound (I) is of formula (IV): 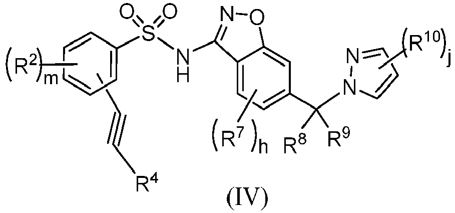 R², R⁴, R⁷, R⁸, R⁹, R¹⁰, m, h and j are as defined above.

In another preferred embodiment, the compound (I) is of formula (V): 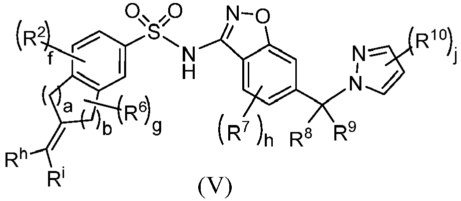 R², R⁶, R⁷, R⁸, R⁹, R¹⁰, R^{h}, Rⁱ, a, b, f, g, h and j are as defined above.

In another preferred embodiment, the compound (I) is of formula (VI): 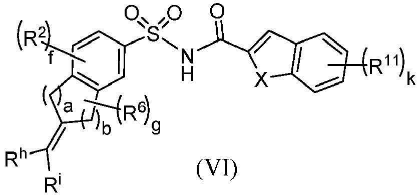 R², R⁶, R¹¹, R^{h}, Rⁱ, X, a, b, f, g and k are as defined above.

In another preferred embodiment, the compound (I) is of formula (VII): 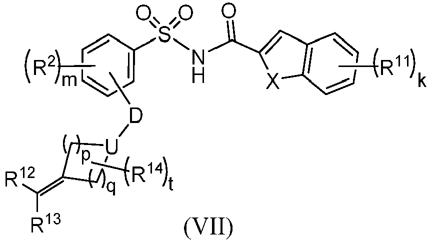
R², R¹¹, R¹², R¹³, D, X, m and k are as defined above;
U, R¹⁴, p, q and t are as defined above.

In another preferred embodiment, the compound (I) is of formula (VIII): 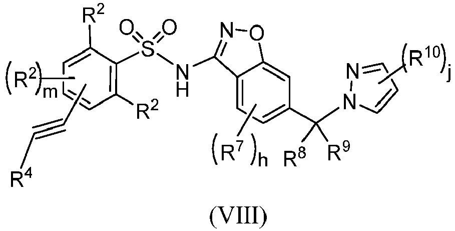
each R² is independently selected from the group consisting of hydrogen, halogen, C₁₋₄ alkyl, C₁₋₄ haloalkyl, C₂₋₄ alkynyl, C₃₋₆ cycloalkyl, CN, OR^{a}, SR^{a}, or NR^{a}R^{a}; wherein each R^{a} is independently selected from hydrogen, C₁₋₄ alkyl, C₁₋₄ haloalkyl, C₃₋₆ cycloalkyl; m is selected from 0, 1 or 2;
R⁴, R⁷, R⁸, R⁹, R¹⁰, h and j are as defined above.

In another preferred embodiment, the R⁴ is selected from the group consisting of hydrogen, C₁₋₄ alkyl, C₃₋₈ cycloalkyl, 3-to 9-membered heterocyclyl, aryl, and heteroaryl; wherein said alkyl, cycloalkyl, heterocyclyl, aryl, or heteroaryl is optionally substituted by one or more groups selected from the group consisting od halogen, CN, OR^{a}, -C(O)R^{m}, -C(O)OR^{a}, -S(O)₂R^{m}, C₁₋₄ alkyl, C₃₋₆ cycloalkyl, 3-to 8-membered heterocyclyl, aryl, and heteroaryl; or said cycloalkyl or heterocyclyl is substituted with =T; wherein each R^{a} is as defined above; R^{m} is selected from the group consisting of C₁₋₄ alkyl, C₂₋₄ alkenyl, C₂₋₄ alkynyl, and C₃₋₆ cycloalkyl; and T is as defined above.

In another preferred embodiment, the R² is each independently selected from the group consisting of hydrogen, halogen, C₁₋₄ alkyl, C₁₋₄ haloalkyl, OR^{a}, C₃₋₆ cycloalkyl, and 3-to 8-membered heterocyclyl.

In another preferred embodiment, the R⁷ is each independently selected from hydrogen, halogen, C₁₋₄ alkyl, C₁₋₄ haloalkyl, OR^{a}, SR^{a}, NR^{a}R^{a}, C₃₋₆ cycloalkyl, 3-to 8-membered heterocyclyl, aryl, heteroaryl, or heteroaryl C₁₋₄ alkyl; each R^{a} is independently selected from hydrogen and C₁₋₄ alkyl.

In another preferred embodiment, the compound (I) is of formula (IX): 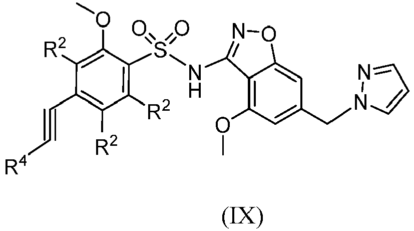
each R² is independently selected from the group consisting of hydrogen, halogen, C₁₋₄ alkyl, C₁₋₄ haloalkyl, C₃₋₆ cycloalkyl, and OR^{a};
R⁴ is selected from the group consisting of hydrogen, C₁₋₄ alkyl, C₃₋₈ cycloalkyl, 3-to 9-membered heterocyclyl, aryl, and heteroaryl; wherein said alkyl, cycloalkyl, heterocyclyl, aryl, or heteroaryl is optionally substituted with one or more groups selected from the group consisting: halogen, CN, OR^{a}, SR^{a}, NR^{a}R^{a}, -C(O)R^{m}, -C(O)OR^{a}, -S(O)₂R^{m}, C₁₋₄ alkyl, C₂₋₄ alkenyl, C₂₋₄ alkynyl, C₃₋₆ cycloalkyl, 3-to 6-membered heterocyclyl, aryl, and heteroaryl; or the cycloalkyl or heterocyclyl is substituted with =T; wherein, T is selected from O or CR¹²R¹³; wherein R¹² and R¹³ are each independently selected from hydrogen, fluorine, and C₁₋₄ alkyl; the alkyl on R¹² or R¹³ is optionally substituted with one or more groups selected from the group consisting of halogen, C₁₋₄ haloalkyl, CN, OR^{b}, SR^{b}, and NR^{b}R^{b};
each R^{a} is independently selected from hydrogen, C₁₋₄ alkyl, C₁₋₄ haloalkyl, and C₃₋₆ cycloalkyl; each R^{b} is independently selected from hydrogen, C₁₋₄ alkyl, and C₁₋₄ haloalkyl; and R^{m} is selected from C₁₋₄ alkyl, C₂₋₄ alkenyl, C₂₋₄ alkynyl and C₃₋₆ cycloalkyl.

In another preferred embodiment, the compound (I) is of formula (X): 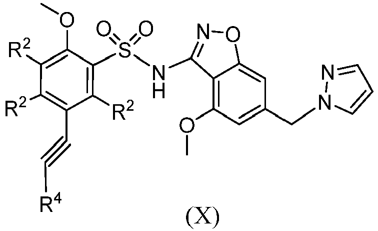
each R² is independently selected from the group consisting of hydrogen, halogen, C₁₋₄ alkyl, C₁₋₄ haloalkyl, C₃₋₆ cycloalkyl, and OR^{a};
R⁴ is selected from the group consisting of hydrogen, C₁₋₄ alkyl, C₃₋₈ cycloalkyl, 3-to 9-membered heterocyclyl, aryl, and heteroaryl; wherein said alkyl, cycloalkyl, heterocyclyl, aryl, or heteroaryl is optionally substituted with one or more groups selected from the group consisting: halogen, CN, OR^{a}, SR^{a}, NR^{a}R^{a}, -C(O)R^{m}, -C(O)OR^{a}, -S(O)₂R^{m}, C₁₋₄ alkyl, C₂₋₄ alkenyl, C₂₋₄ alkynyl, C₃₋₆ cycloalkyl, 3-to 6-membered heterocyclyl, aryl, and heteroaryl; or the cycloalkyl or heterocyclyl is substituted with =T; wherein, T is selected from O and CR¹²R¹³; wherein R¹² and R¹³ are each independently selected from hydrogen, fluorine, or C₁₋₄ alkyl; the alkyl on R¹² or R¹³ is optionally substituted with one or more groups selected from the group consisting of halogen, C₁₋₄ haloalkyl, CN, OR^{b}, SR^{b}, and NR^{b}R^{b};
each R^{a} is independently selected from hydrogen, C₁₋₄ alkyl, C₁₋₄ haloalkyl, and C₃₋₆ cycloalkyl; each R^{b} is independently selected from hydrogen, C₁₋₄ alkyl, and C₁₋₄ haloalkyl; and R^{m} is selected from C₁₋₄ alkyl, C₂₋₄ alkenyl, C₂₋₄ alkynyl and C₃₋₆ cycloalkyl.

In another preferred embodiment, the compound (I) is of formula (IX): 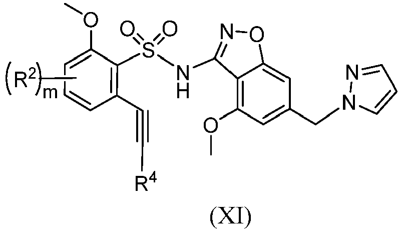
each R² is independently selected from the group consisting of hydrogen, halogen, C₁₋₄ alkyl, C₁₋₄ haloalkyl, C₁₋₄ alkoxy-C₁₋₄ alkyl, C₁₋₄ haloalkoxy-C₁₋₄ alkyl, C₂₋₄ alkenyl, C₂₋₄ haloalkenyl, C₂₋₄ alkynyl, C₃₋₆ cycloalkyl, 3-to 8-membered heterocyclyl, aryl, heteroaryl, CN, OR^{a}, SR^{a}, and NR^{a}R^{a};
R⁴ is selected from the group consisting of hydrogen, C₁₋₄ alkyl, and C₃₋₆ cycloalkyl; wherein the alkyl or cycloalkyl is optionally substituted with one or more groups selected from the group consisting of halogen, CN, OR^{a}, SR^{a} and NR^{a}R^{a};
each R^{a} is independently selected from hydrogen, C₁₋₄ alkyl, C₁₋₄ haloalkyl and C₃₋₆ cycloalkyl;
m is selected from 0, 1, 2 and 3.

In another preferred embodiment, the compound (I) is of formula (XII): 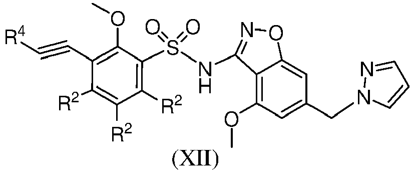 R² and R⁴ are as defined above.

In another preferred embodiment, the compound (I) is of formula (VII): 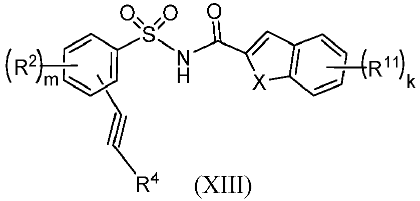 R², R⁴, R¹¹, X, m and k are as defined above.

In the second aspect of the present invention, a compound of the structure shown in formula (XIV), or an optical isomer, a pharmaceutically acceptable salt, a prodrug, a deuterated derivative, a hydrate, or a solvent thereof is provided: 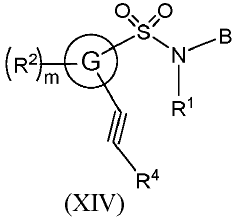
G is selected from C₃₋₈ cycloalkyl, 3-to 10-membered heterocyclyl, aryl (preferably benzene, naphthalene), and heteroaryl (preferably furan, thiophene, pyridine, pyrimidine, pyrazine);
R¹, R², R⁴, B, and m are as defined above.

In another preferred embodiment, the compound of formula (I) or formula (XIV) is selected from the group consisting of 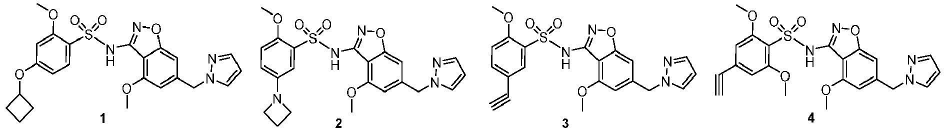 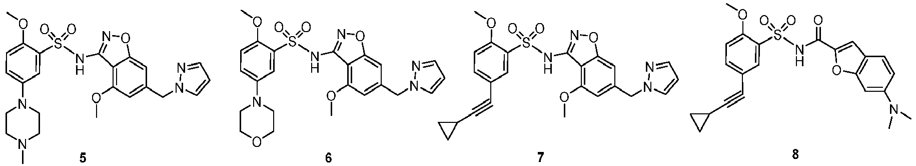 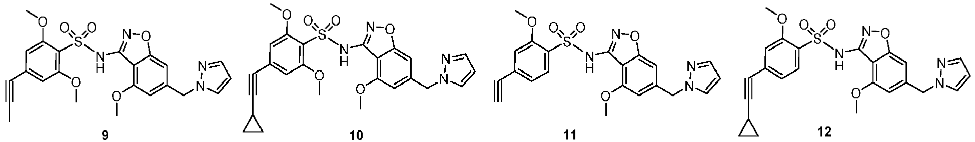 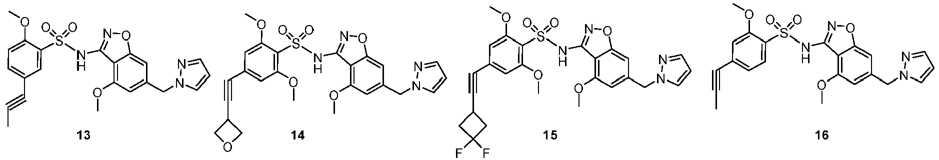 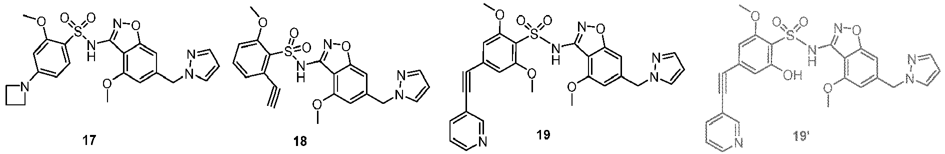 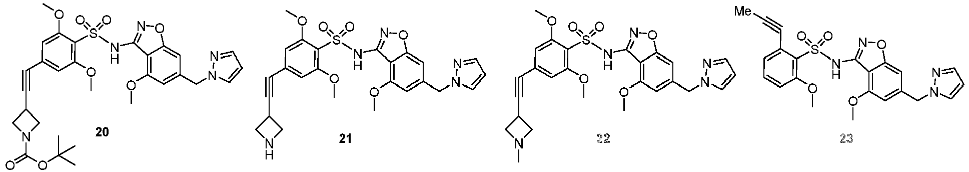 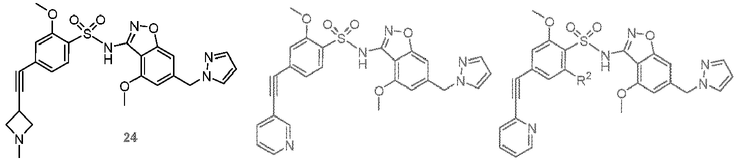 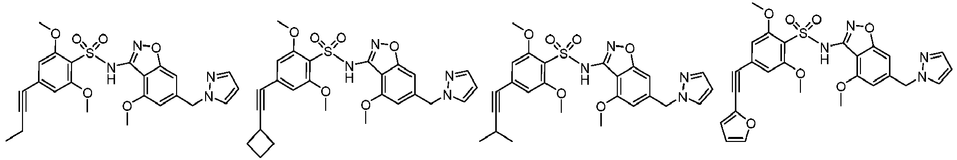 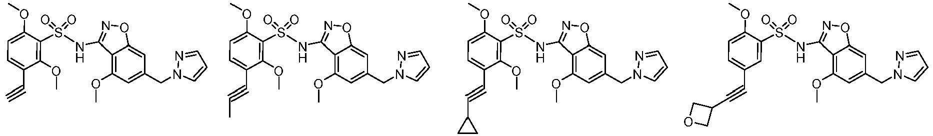 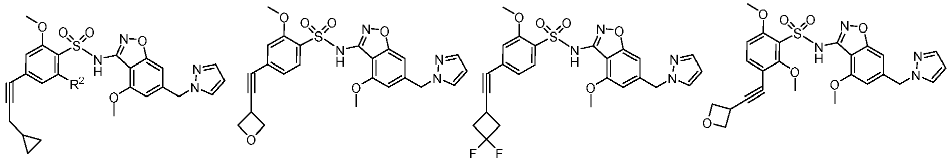 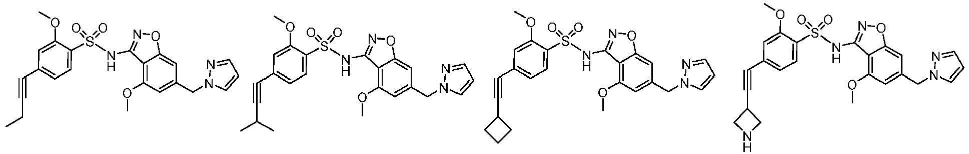 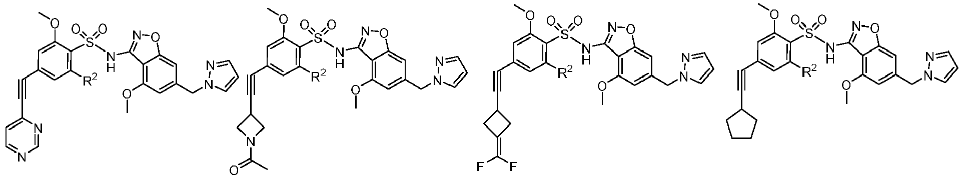 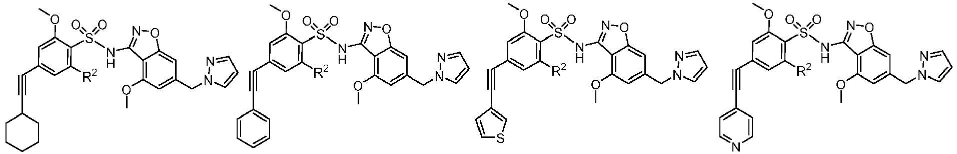 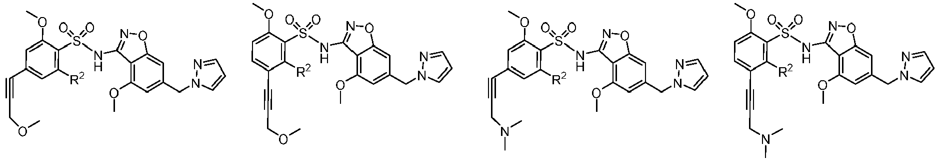 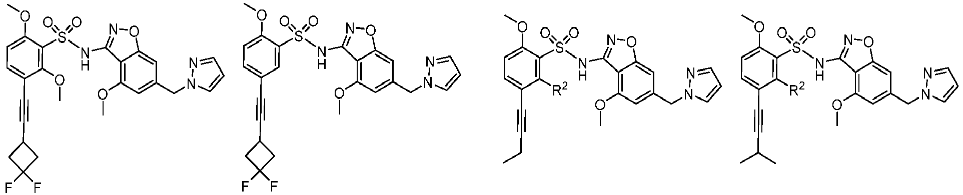 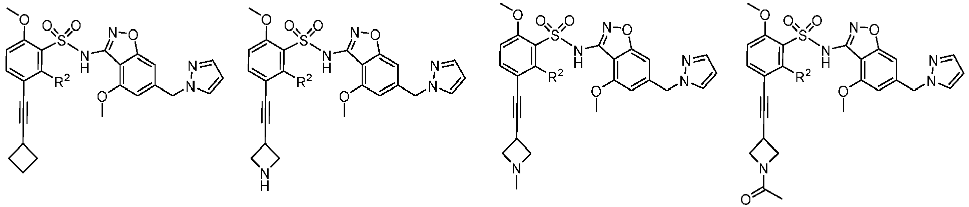 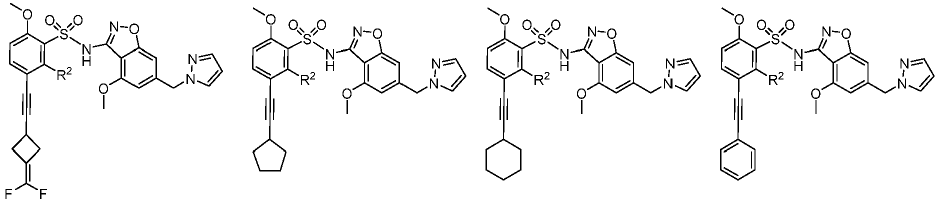 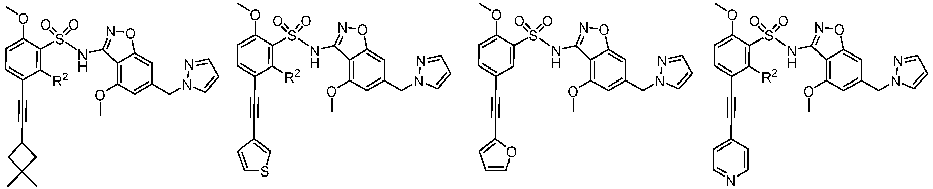 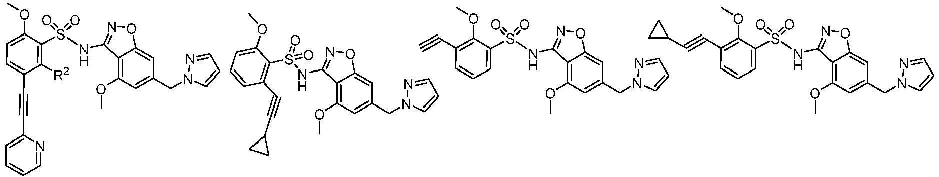 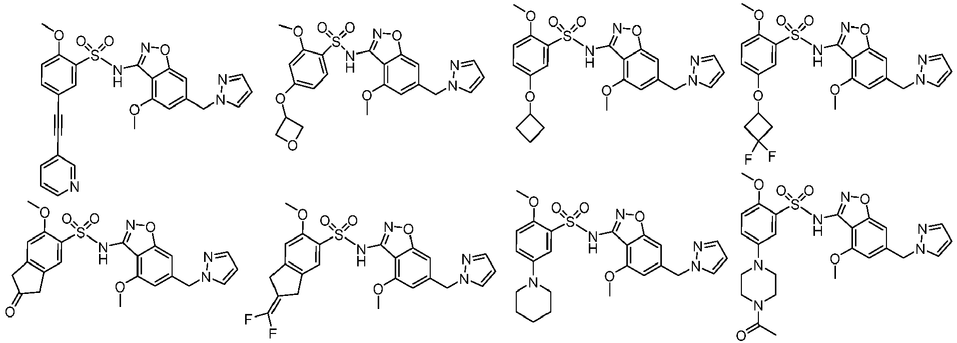 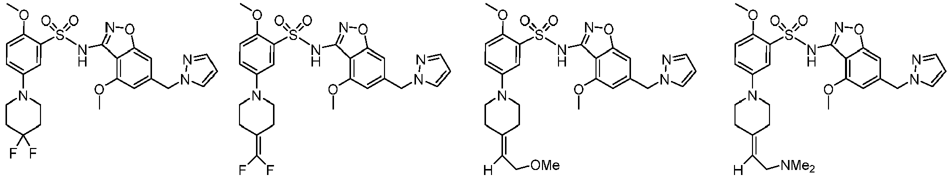 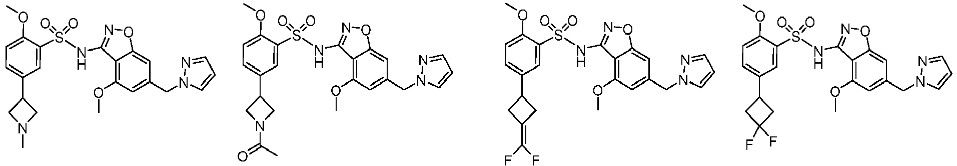 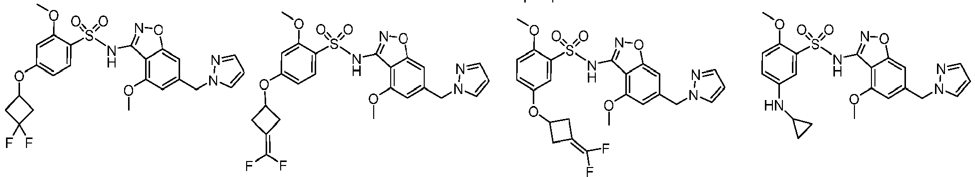 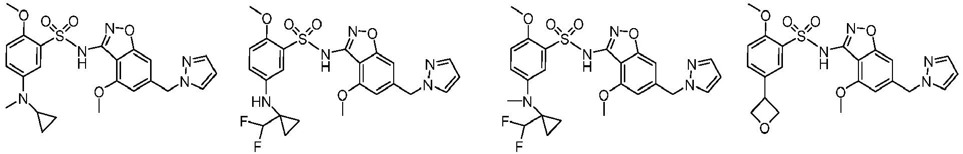 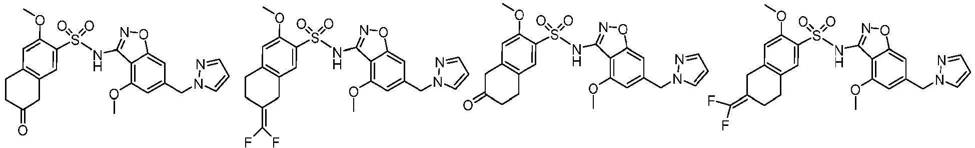 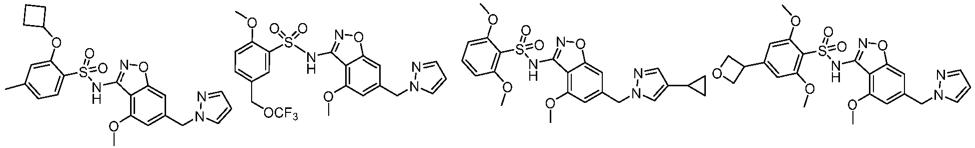 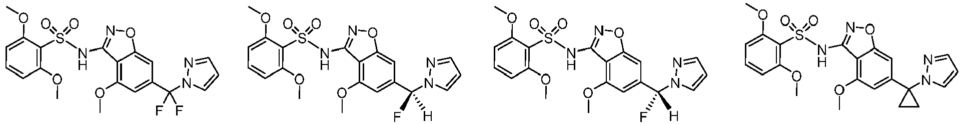 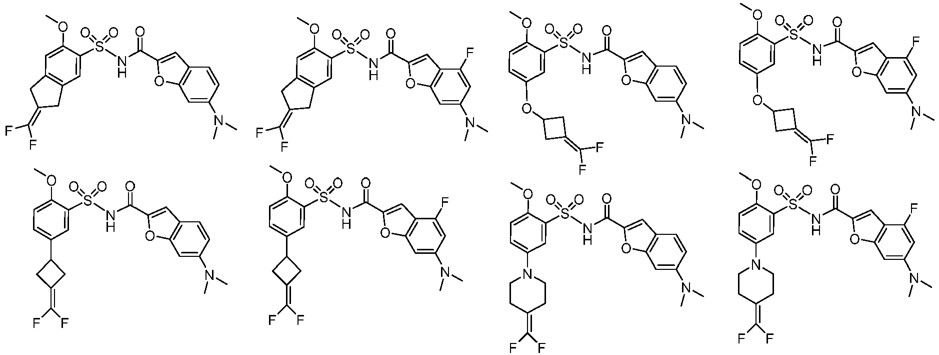 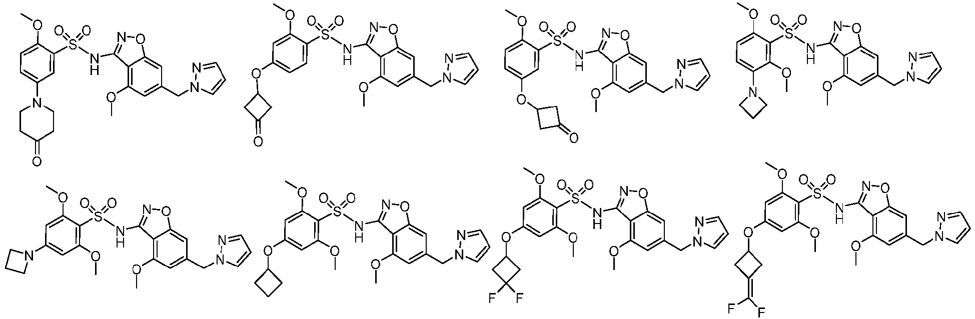 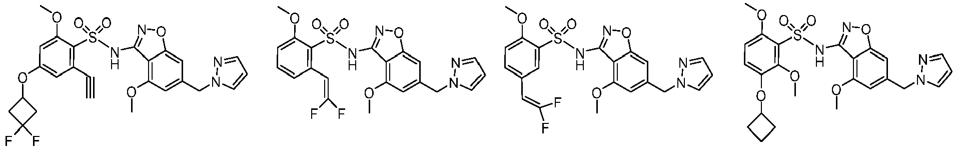 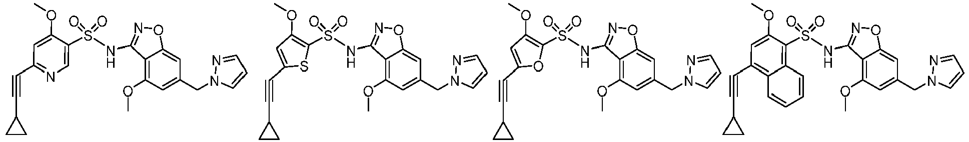 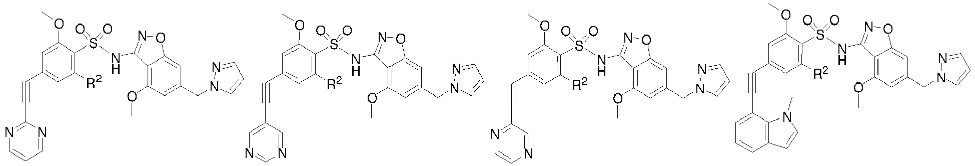 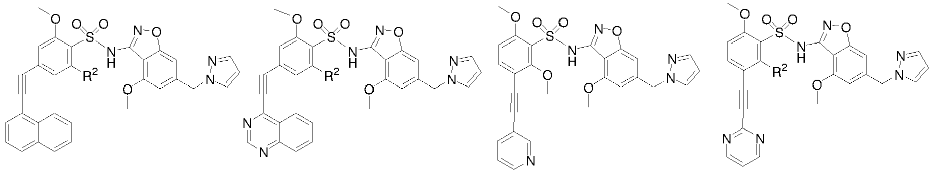 in each of the above compounds, R² is selected from H or OMe.

In the third aspect of the present invention, a pharmaceutical composition, wherein comprises the compound of the first aspect of the present invention, or the optical, tautomer, or a pharmaceutically acceptable salt, hydrate or solvate thereof, and (2) pharmaceutically acceptable carriers is provided.

In the fourth aspect of the present invention, a use of the compound of the first aspect of the present invention, or an optical isomer, a pharmaceutically acceptable salt, a prodrug, a deuterated derivative, a hydrate, or a solvent compound thereof in the preparation of a pharmaceutical composition for the treatment of a disease, condition, or condition associated with KAT6 activity or expression.

In another preferred embodiment, said disease, condition or disease is selected from the group consisting of non-small cell lung cancer, small cell lung cancer, adenocarcinoma of the lung, squamous lung cancer, pancreatic cancer, colon cancer, thyroid cancer, embryonal rhabdomyosarcoma, granulosa cell tumor of the skin, melanoma, hepatocellular carcinoma, intrahepatic cholangiocarcinoma, rectal carcinoma, bladder carcinoma, pharyngolaryngeal cancer, breast carcinoma, vaginal carcinoma, prostate cancer, testicular cancer, brain tumor, glioblastoma, ovarian cancer, head and neck squamous cell carcinoma, cervical cancer, osteosarcoma, esophageal cancer, kidney cancer, skin cancer, gastric cancer, myeloid leukemia, lymphatic leukemia, myeloid fibrosis, B-cell lymphoma, T-cell lymphoma, Hodgkin's lymphoma, non-Hodgkin's lymphoma, monocytic leukemia, polycythemia megalosplenica, Eosinophilic leukocytosis syndrome multiple, bone marrow cancer, and various other solid and hematologic tumors, etc..

### Detailed Implementation

After long-term and intensive research, the present inventors have unexpectedly discovered a KAT6 protein kinase inhibitor of novel structure, as well as their preparation methods and applications. The compounds of the invention may be applied to the treatment of various diseases associated with the activity of acetyltransferase kinases. Based on the above findings, the inventors completed the present invention.

### Terminology

Unless otherwise stated, "or" as used herein has the same meaning as "and/or" (refers to "or" and "and").

Unless otherwise specified, among all compounds of the present invention, each chiral carbon atom (chiral center) may optionally be in the R configuration or the S configuration, or a mixture of the R configuration and the S configuration.

As used herein, the term "alkyl", alone or as part of another substituent, refers to a straight (ie, unbranched) or branched saturated hydrocarbon group containing only carbon atoms, or a combination of straight and branched chains. When the alkyl group has a carbon number limitation (e.g., C₁₋₁₀), it means that the alkyl group has 1 to 10 carbon atoms. For example, C₁₋₈ alkyl refers to an alkyl group containing from 1 to 8 carbon atoms, including methyl, ethyl, propyl, isopropyl, butyl, isobutyl, sec-butyl, tert-butyl, or the like.

As used herein, the term "alkenyl", when used alone or as part of another substituent, refers to a straight or branched, carbon chain group having at least one carbon-carbon double bond. Alkenyl groups can be substituted or unsubstituted. When the alkenyl group has a carbon number limit (e.g., C₂₋₈), it means that the alkenyl group has 2-8 carbon atoms. For example, C₂₋₈ alkenyl refers to alkenyl groups having 2-8 carbon atoms, including ethenyl, propenyl, 1,2-butenyl, 2,3-butenyl, butadienyl, or the like.

As used herein, the term "alkynyl", when used alone or as part of another substituent, refers to an aliphatic hydrocarbon group having at least one carbon-carbon triple bond. The alkynyl group can be straight or branched, or a combination thereof. When the alkynyl group has a carbon number limitation (e.g., C₂₋₈ alkynyl group), it means that the alkynyl group has 2 to 8 carbon atoms. For example, the term "C₂₋₈ alkynyl" refers to a straight or branched alkynyl group having 2-8 carbon atoms, including ethynyl, propynyl, isopropynyl, butynyl, isobutynyl, secondary Butynyl, tert-butynyl, or the like.

As used herein, when used alone or as part of another substituent, the term "cycloalkyl" refers to a group having a saturated or partially saturated ring, a bicyclic or polycyclic (fused ring, bridged or spiro) ring system. When a certain cycloalkyl group has a carbon number limitation (e.g., C₃₋₁₀), it means that the cycloalkyl group has 3 to 10 carbon atoms. In some preferred embodiments, the term "C3-8 cycloalkyl" refers to a saturated or partially saturated monocyclic or bicyclic alkyl group having from 3 to 8 carbon atoms, including cyclopropyl, cyclobutyl, cyclopentyl, cycloheptyl, or the like. "Spirocycloalkyl" refers to a bicyclic or polycyclic group that shares a carbon atom (called a spiro atom) between the monocyclic rings. These may contain one or more double bonds, but none of the rings have fully conjugated π electrons system. "Fused cycloalkyl" refers to an all-carbon bi-cyclic or polycyclic group in which each ring share two neighboring carbon atoms with other ring(s), which may contain one or more double bonds, but none of the rings have a fully conjugated π-electron system. "Bridge cycloalkyl" refers to an all-carbon polycyclic group in which two rings share two carbon atoms that are not directly bonded, which may contain one or more double bonds, but none of the rings have a fully conjugated π-electron system. The atoms contained in the cycloalkyl group are all carbon atoms. Some examples of cycloalkyl groups are as follows, and the present invention is not limited to the following cycloalkyl groups.

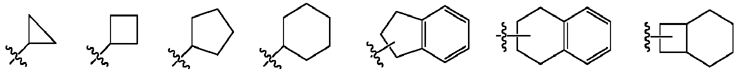

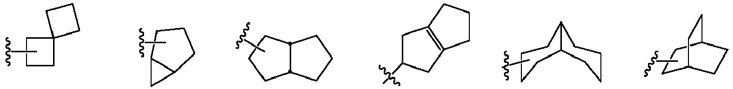

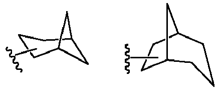

Unless otherwise stated, the following terms used in the specification and claims have the following meanings. "Aryl" means an all-carbon monocyclic or fused polycyclic (ie, a ring that shares a pair of adjacent carbon atoms) groups having a conjugated π-electron system, such as phenyl and naphthyl. The aryl ring may be fused to other cyclic groups (including saturated and unsaturated rings), but may not contain heteroatoms such as nitrogen, oxygen, or sulfur, while the point of attachment to the parent must be on the carbon atoms of the ring in a conjugated π-electron system. The aryl group can be substituted or unsubstituted. The following are some examples of aryl groups, and the present invention is not limited to the aryl groups described below.

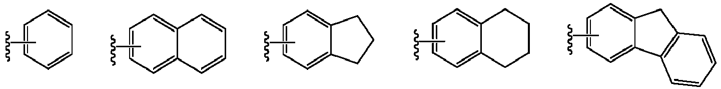

"Heteroaryl" refers to an aromatic monocyclic or polycyclic group containing one to more heteroatoms (optionally from nitrogen, oxygen, and sulfur), or a polycyclic group formed by condensing a heterocyclic group (containing one to more heteroatoms, optionally selected from nitrogen, oxygen, and sulfur) with an aryl group, and the attachment site is located on the aryl group. The heteroaryl group can be optionally substituted or unsubstituted. The following are some examples of heteroaryl groups, and the present invention is not limited to the following heteroaryl groups.

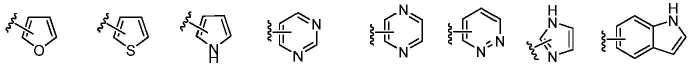

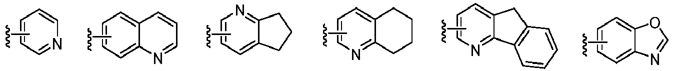

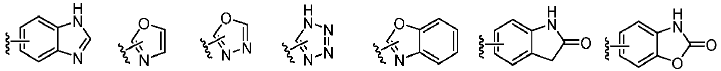

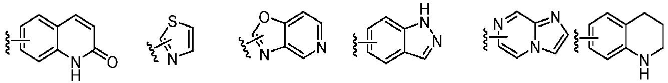

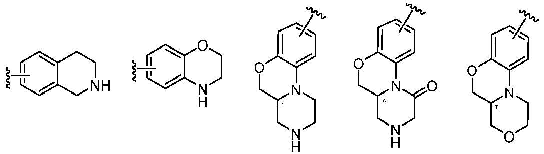

"Heterocyclyl" means a saturated or partially unsaturated monocyclic or polycyclic cyclic hydrocarbon substituent wherein one or more of the ring atoms are selected from nitrogen, oxygen or sulfur and the remaining ring atoms are carbon. Non-limiting examples of monocyclic heterocyclic groups include pyrrolidinyl, piperidinyl, piperazinyl, morpholinyl, thiomorpholinyl, homopiperazinyl. Polycyclic heterocyclic group refers to a heterocyclic group including a spiro ring, a fused ring, and a bridged ring. "Spirocyclic heterocyclyl" refers to a polycyclic heterocyclic group in which each ring of the system shares an atom (referred to as a spiro atom) with other rings in the system, wherein one or more of the ring atoms is selected from the group consisting of nitrogen and oxygen. Or sulfur, the remaining ring atoms are carbon. "Fused ring heterocyclyl" refers to a polycyclic heterocyclic group in which each ring of the system shares an adjacent pair of atoms with other rings in the system, and one or more rings may contain one or more double bonds, but none One ring has a fully conjugated pi-electron system, and wherein one or more ring atoms are selected from nitrogen, oxygen or sulfur, and the remaining ring atoms are carbon. "Bridged heterocyclyl" refers to a polycyclic heterocyclic group in which any two rings share two atoms which are not directly bonded, these may contain one or more double bonds, but none of the rings have a fully conjugated pi-electron system, and wherein one or more of the ring atoms are selected from nitrogen, oxygen or sulfur, and the remaining ring atoms are carbon. If a heterocyclic group has both a saturated ring and an aromatic ring (for example, the saturated ring and the aromatic ring are fused together), the point attached to the parent must be on the saturated ring. Note: When the point attached to the parent is on the aromatic ring, it is called a heteroaryl group and is not called a heterocyclic group. Some examples of the heterocyclic group are as follows, and the present invention is not limited to the following heterocyclic group.

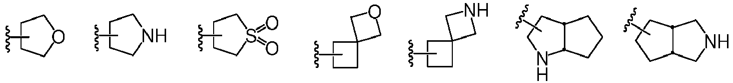

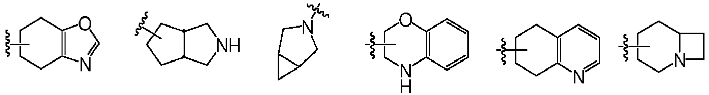

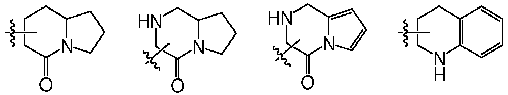

As used herein, the term "halogen", when used alone or as part of another substituent, refers to F, Cl, Br, and I.

As used herein, the term "substituted" (when with or without "optionally") means that one or more hydrogen atoms on a particular group are replaced by a particular substituent. Particular substituents are the substituents described above in the corresponding paragraphs, or the substituents which appear in the examples. Unless otherwise stated, an optionally substituted group may have a substituent selected from a particular group at any substitutable position of the group, and the substituents may be the same or different at each position. A cyclic substituent, such as a heterocyclic group, may be attached to another ring, such as a cycloalkyl group, to form a spirobicyclic ring system, i.e., the two rings have a common carbon atom. Those skilled in the art will appreciate that the combinations of substituents contemplated by the present invention are those that are stable or chemically achievable. The substituents are, for example but not limited to, C₁₋₈ alkyl, alkenyl, alkynyl, C₃₋₈ cycloalkyl, 3- to 12-membered heterocyclyl, aryl, heteroaryl, halogen, hydroxy, carboxy (-COOH), C₁₋₈ aldehyde, C₂₋₁₀ acyl, C₂₋₁₀ ester, amino group.

For convenience and in accordance with conventional understanding, the term "optionally substituted" or "optionally substituted" applies only to sites which are capable of being substituted by a substituent, and does not include those which are not chemically achievable.

As used herein, unless otherwise specified, the term "pharmaceutically acceptable salt" refers to a salt that is suitable for contact with the tissue of a subject (eg, a human) without causing unpleasant side effects. In some embodiments, a pharmaceutically acceptable salt of a compound of the invention includes a salt (eg, a potassium salt, a sodium salt, a magnesium salt, a calcium salt) of a compound of the invention having an acidic group or is basic A salt of a compound of the invention (e.g., a sulfate, a hydrochloride, a phosphate, a nitrate, a carbonate).

### Application:

The present invention provides a class of compounds of formula (I), or their deuterated derivatives, their pharmaceutically acceptable salts, optical isomers (enantiomers or diastereomers, if any case), hydrates, solvates, or pharmaceutical combinations comprising the compound represented by formula (I), its optical isomers, pharmaceutically acceptable salts, prodrugs, deuterated forms, hydrates, and solvates for inhibiting KAT6 kinase activity.

The compound of the present invention can be used as KAT6 kinase inhibitor.

The present invention is a single inhibitor of KAT6 for the purpose of preventing, alleviating or curing a disease by modulating the activity of KAT6. The referred diseases include, but are not limited to: non-small cell lung cancer, small cell lung cancer, lung adenocarcinoma, squamous lung cancer, pancreatic cancer, colon cancer, thyroid cancer, embryonal rhabdomyosarcoma, granular cell tumor of the skin, melanoma, hepatocellular carcinoma, intrahepatic cholangiocarcinoma, rectal carcinoma, bladder cancer, pharyngeal carcinoma, breast carcinoma, vaginal carcinoma, prostate carcinoma, testicular carcinoma, brain tumor, glioma, ovarian carcinoma, squamous cancer of the head and neck, Cervical Cancer, Osteosarcoma, Esophageal Cancer, Kidney Cancer, Skin Cancer, Gastric Cancer, Myeloid Leukemia, Lymphoid Leukemia, Myelofibrosis, B-cell Lymphoma, T-cell Lymphoma, Hodgkin's Lymphoma, Non-Hodgkin's Lymphoma, Mononuclear Cell Leukemia, Erythropoietic Eosinophilic Leukocytosis, Eosinophilic Leukocytosis Syndrome Multi-Cellular, Myeloid Carcinoma and other solid and hematologic tumors.

The compounds of the present invention and their deuterated forms, as well as pharmaceutically acceptable salts or isomers (if present) or hydrates and/or compositions thereof can be combined with pharmaceutically acceptable excipients or carriers formulated together, the resulting composition can be administered to humans or animals for the treatment of disorders, symptoms and diseases. The composition can be: tablets, pills, suspensions, solutions, emulsions, capsules, aerosols, sterile injections, sterile powders and the like. In a preferred embodiment, the pharmaceutical composition is a dosage form suitable for oral administration, including but not limited to tablets, solutions, suspensions, capsules, granules, and powders. The amount of the compound or pharmaceutical composition administered to the patient is not fixed, and is usually administered in a pharmaceutically effective amount. At the same time, the amount of the compound actually administered can be determined by the physician according to the actual situation, including the disease to be treated, the route of administration selected, the actual compound administered, the individual condition of the patient, and so on. The dosage of the compound of the present invention depends on the specific use of the treatment, the mode of administration, the state of the patient, and the judgment of the physician. The ratio or concentration of the compound of the present invention in the pharmaceutical composition depends on a variety of factors, including dosage, physical and chemical properties, route of administration, and the like.

It is to be understood that within the scope of the present invention, the various technical features of the present invention and the various technical features specifically described hereinafter (as in the embodiments) may be combined with each other to form a new or preferred technical solution.

### Pharmaceutical composition and method of administration

Since the compound of the present invention has excellent inhibitory activity against KAT6, the compound of the present invention and its various crystal forms, optical isomers, pharmaceutically acceptable inorganic or organic salts, prodrugs, deuterated forms, hydrates or solvates, and pharmaceutical compositions containing the compounds of the present invention as the main active ingredients can be used to treat, prevent and alleviate diseases associate with the activity or expression of KAT6.

The pharmaceutical compositions of the present invention comprise a safe or effective amount of a compound of the present invention, or a pharmaceutically acceptable salt thereof, and a pharmaceutically acceptable excipient or carrier. By "safe and effective amount" it is meant that the amount of the compound is sufficient to significantly improve the condition without causing serious side effects. In general, the pharmaceutical compositions contain from 1 to 2000 mg of the compound of the invention per agent, more preferably from 5 to 200 mg of the compound of the invention per agent. Preferably, the "one dose" is a capsule or tablet.

"Pharmaceutically acceptable carrier" means: one or more compatible solid or liquid fillers or gel materials which are suitable for human use and which must be of sufficient purity and of sufficiently low toxicity. By "compatibility" it is meant herein that the components of the composition are capable of intermingling with the compounds of the invention and with each other without significantly reducing the efficacy of the compound. Examples of pharmaceutically acceptable carriers include, but are not limited to, filler (or diluent), disintegrant, lubricant, binder, matrix, emulsifiers, run wet agents, colorants, flavoring agents, stabilizers, antioxidants, preservatives, pyrogen-free water, and the like.

The mode of administration of the compound or pharmaceutical composition of the present invention is not particularly limited, and representative modes of administration include, but are not limited to, oral, intratumoral, rectal, parenteral (intravenous, intramuscular or subcutaneous), and topical administration.

Solid dosage forms for oral administration include capsules, tablets, pills, powders, and granules. In these solid dosage forms, the active compound is mixed with at least one conventional inert excipient (or carrier). In capsules, tablets and pills, the dosage form may also contain a buffer.

Solid dosage forms such as tablets, dragees, capsules, pills, and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric and waxy materials. If necessary, the active compound may also be in microencapsulated form with one or more of the above-mentioned excipients.

In addition to these inert diluents, the compositions may contain adjuvants such as wetting agents, emulsifying and suspending agents, sweetening agents, flavoring agents and perfumes.

In addition to the active compound, the suspension may contain suspending agents.

Compositions for parenteral injection may comprise a physiologically acceptable sterile aqueous or nonaqueous solution, dispersion, suspension or emulsion, and a sterile powder for reconstitution into a sterile injectable solution or dispersion. Suitable aqueous and nonaqueous vehicles, diluents, solvents or vehicles include water, ethanol, polyols, and suitable mixtures thereof.

Dosage forms for the compounds of the invention for topical administration include ointments, powders, patches, propellants and inhalants. The active ingredient is admixed under sterile conditions with a physiologically acceptable carrier and any preservatives, buffers, or, if necessary, propellants.

The compounds of the invention may be administered alone or in combination with other pharmaceutically acceptable compounds.

When a pharmaceutical composition is used, a safe and effective amount of a compound of the invention is administered to a mammal (e.g., a human) in need of treatment wherein the dosage is a pharmaceutically effective effective dosage, for a 60 kg body weight, The dose to be administered is usually from 1 to 2000 mg, preferably from 5 to 500 mg. Of course, specific doses should also consider factors such as the route of administration, the health of the patient, etc., which are within the skill of the skilled physician.

### The main advantages of the present invention include:

1. Provided a compound as shown in Formula I.
2. Provided a structurally novel KAT6 inhibitor, and the preparation and application thereof, said inhibitor being able to inhibit the activity of KAT6 at a very low concentration.
3. Provided a KAT6 inhibitor of good oral absorption.
4. Provided a class of pharmaceutical compositions for the treatment of diseases associated with KAT6 activity.

The invention is further illustrated below in conjunction with specific embodiments. It is to be understood that the examples are not intended to limit the scope of the invention. The experimental methods in the following examples which do not specify the specific conditions are usually in accordance with conventional conditions or according to the conditions recommended by the manufacturer. Percentages and parts are by weight unless otherwise stated.

Some of the representative compounds of the present invention can be prepared by the following synthetic methods, and in each of the following reaction formulas, the reagents and conditions for each step can be selected from those conventional in the field for carrying out such preparation methods, and the above selections can be made by a person skilled in the art according to the knowledge in the field after the structures of the compounds of the present invention are disclosed.

### General synthetic methods for compounds

Some compounds of the present invention can be prepared by the following methods:

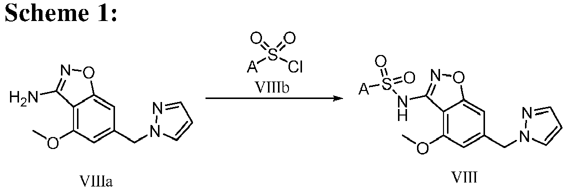

Compound (Villa) can be synthesized by using the method described in patent WO2020254946, while compound (VIIIb) can be synthesized using relevant references. In an inert solvent, compound (Villa) reacts with compound (VIIIb) to obtain compound (VIII). The definition of A in the above reaction formula is the same as the description in claim 1.

The compound of formula (II) of the present invention can be prepared by the following methods:

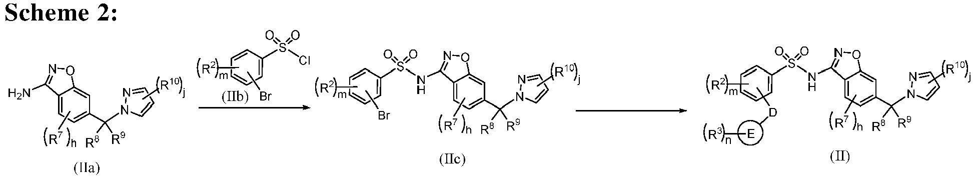

Compound (IIa) can be synthesized using the method described in patent WO2020254946. In an inert solvent, compound (IIa) is reacted with compound (IIb) to obtain compound (IIc), which is then combined with the corresponding intermediate or reagent through a metal catalyzed coupling reaction or substitution reaction to synthesize the target compound (II). The definitions of R², R³, R⁷, R⁸, R⁹, R¹⁰, n, m, h, j, D and E in the above reaction formulas are the same as those in the claims.

The compound of formula (III) of the present invention can be prepared by the following methods:

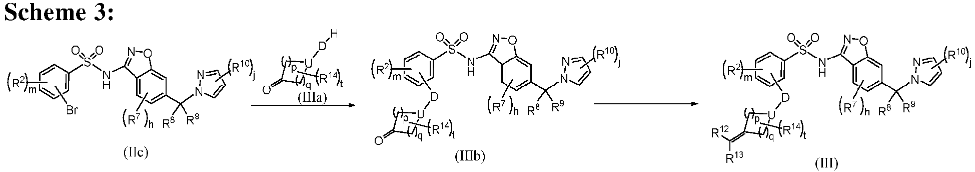

In an inert solvent, the target compound (IIIb) is synthesized by metal catalyzed coupling reaction or substitution reaction between compound (IIc) and compound (IIIa). Compound (III) is obtained through wittig reaction with compound (IIIb). The definitions of R², R⁷, R⁸, R⁹, R¹⁰, R¹², R¹³, R¹⁴, p, q, h, j, m, t, D and U in the above reaction formulas are the same as those in the claims.

The compound of formula (IV) of the present invention can be prepared by the following methods:

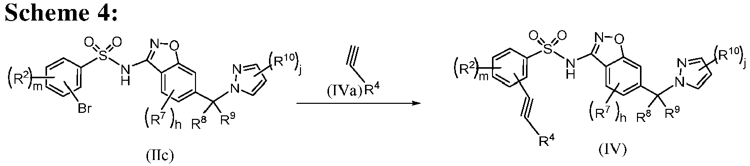

Compound (IV) is obtained by metal catalyzed coupling reaction between compound (IIc) and compound (IVa) in an inert solvent. The definitions of R², R⁴, R⁷, R⁸, R⁹, R¹⁰, h, j, and m in the above reaction formulas are the same as those in the claims.

The compound of formula (V) of the present invention can be prepared by the following methods:

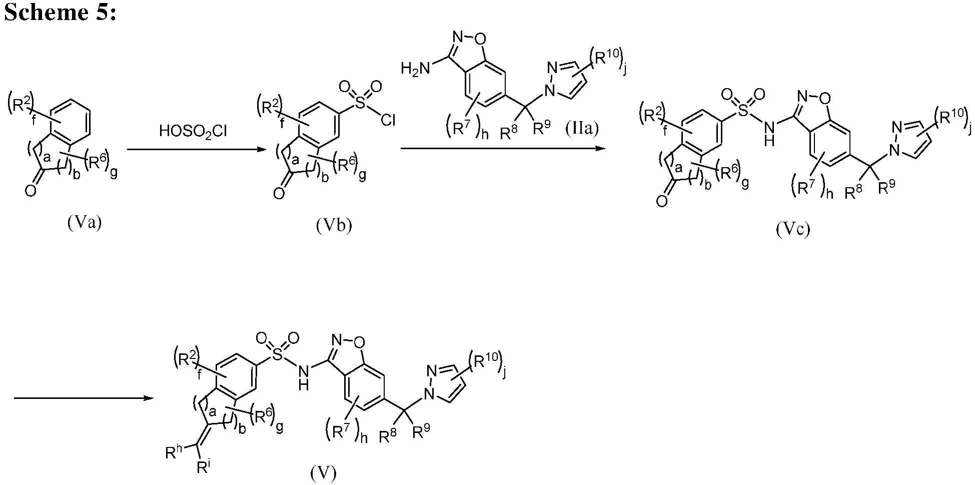

In an inert solvent, compound (Va) reacts with chlorosulfonic acid to obtain compound (Vb). Compound (Vb) reacts with compound (IIa) to obtain compound (Vc), which undergoes a wittig reaction to obtain compound (V). The definitionss of R², R⁶, R⁷, R⁸, R⁹, R¹⁰, R^{h}, Rⁱ, a, b, f, g, h, and j in the above reaction formulas are the same as those in the claims.

The compound of formula (VI) of the present invention can be prepared by the following methods:

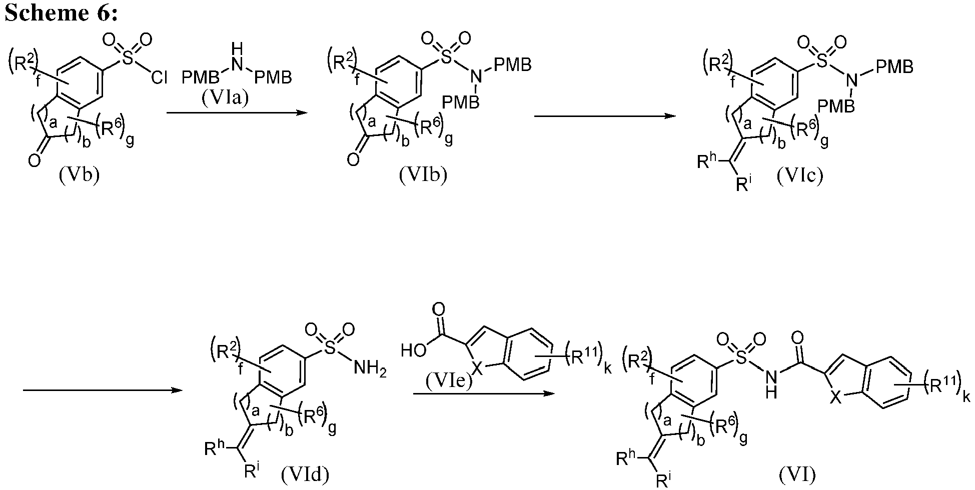

In an inert solvent, compound (Vb) reacts with compound (VIa) to obtain compound (VIb), which is then subjected to a Wittig reaction to obtain compound (VIc). The protective group is removed to obtain compound (VId). The compound (VIe) was synthesized using the method described in patent WO2020216701. Compound (VId) reacts with compound (VIe) to obtain compound (VI). The definitions of R², R⁶, R¹¹, R^{h}, Rⁱ, X, a, b, g, f, and k in the above reaction formulas are the same as those in the claims.

The compound of formula (VII) of the present invention can be prepared by the following method:

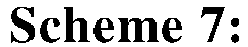

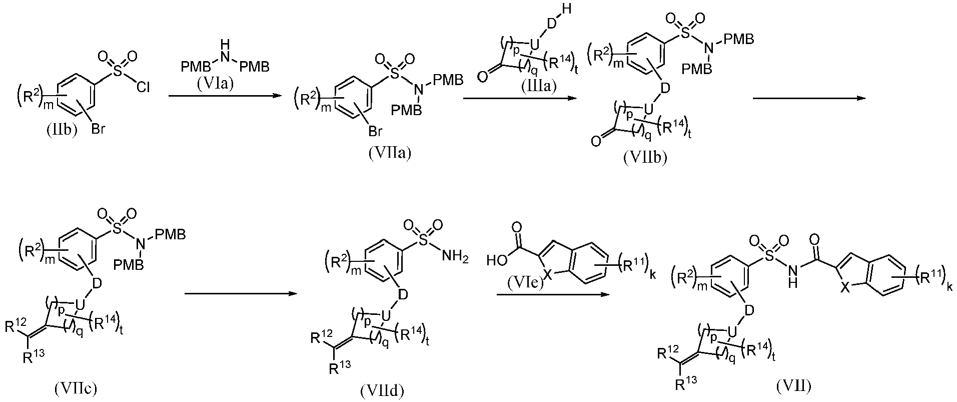

In an inert solvent, compound (IIb) is reacted with compound (VIa) to obtain compound (VIIa), which is then subjected to a metal catalyzed coupling or substitution reaction to obtain compound (VIIb). Compound (VIIb) is then subjected to a Wittig reaction to obtain compound (VIIc), and the protective group is removed to obtain compound (VIId). The compound (VIe) was synthesized using the method described in patent WO2020216701. Compound (VIId) reacts with compound (VIe) to obtain compound (VII). The definitions of R², R¹¹, R¹², R¹³, R¹⁴, X, D, U, p, q, t, m, and k in the above reaction formulas are the same as those in the claims.

### Examples

### Example 1: Preparation of Compound 1

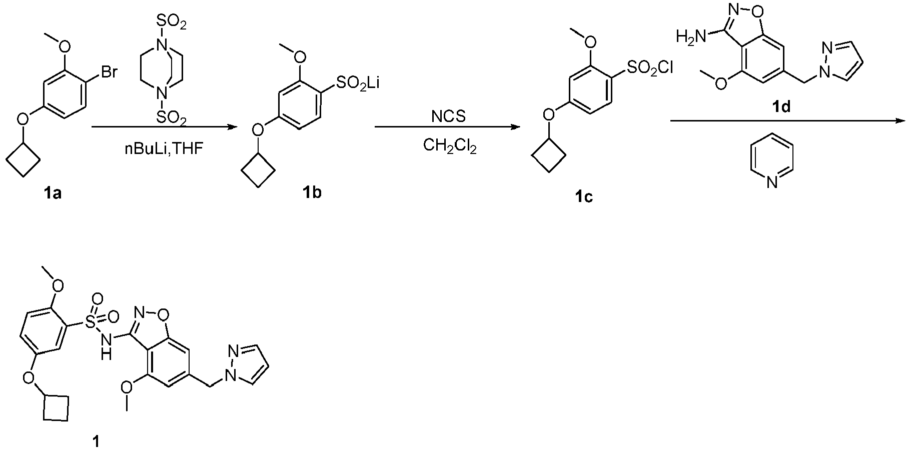

The compound **1a** was synthesized according to WO20130158004. The compound **1a** (205 mg, 0.80 mmol) was dissolved in THF (Tetrahydrofuran, 10 mL). The mixture was cooled to -78 °C and nBuLi (2.5 M in hexane, 0.35 mL) was added. The reaction mixture was stirred at this temperature for 1 h. Then Bis (sulfur dioxide) -1,4-diazabicyclo [2.2.2] octane adduct (115 mg, 0.48 mmol) was added. The mixture was warmed to RT (room temperature) and stirred for 1 h. The reaction mixture was concentrated. The crude was washed by methyl tert-butyl ether (MTBE) and filterated to give solid compound **1b** (180 mg, 91%). The product was used to the next step directly.

The compound **1b** (203 mg, 0.82 mmol) was dissolved in dichloromethane (DCM, 5 mL). Then Chlorobutyrylimide (109 mg, 0.82 mmol) was added under ice bath. The reaction mixture was stirred at this temperature for 1 h. The reaction mixture was filterated and concentrated under reduced pressure. The crude was purified by column chromatography to give white solid compound **1c** (94 mg, yield 42%). ¹H NMR (500 MHz, CDCl₃) δ = 7.78 (d, J = 9.0 Hz, 1H), 6.41 (d, *J* = 2.0 Hz, 1H), 6.36 (dd, *J* = 9.0 Hz, *J* = 2.0 Hz, 1H), 4.68-4.62 (m, 1H), 2.44-2.38 (m, 2H), 2.17-2.10 (m, 2H), 1.89-1.80 (dd, *J*=21.0, 10.4, 1H), 1.72-1.62 (m, 1H) ppm

The compound **Id** was synthesized according to WO2020254946. The compound **1c** (30 mg, 0.11 mmol) was dissolved in pyrdine (0.2 mL). Then **Id** (13 mg, 0.05 mmol) was added. The reaction mixture was stirred at 120 °C for 2 h in a sealed tube. The reaction was concentrated under reduced pressure. The crude was purified by Pre-TLC (Preparative TLC) to give white solid compound **1** (5 mg, yield 20%). ¹H NMR (500 MHz, CDCl₃) δ 8.05-7.94 (m, 2H), 7.60 (s, 1H), 7.46 (s, 1H), 6.79 (s, 1H), 6.50 (s, 1H), 6.42 (dd, *J* = 8.8, 1.8 Hz, 1H), 6.37-6.31 (m, 2H), 5.41 (s, 2H), 4.69-4.61 (m, 1H), 3.96 (s, 3H), 3.84 (s, 3H), 2.48-2.38 (m, 2H), 2.19-2.08 (m, 2H), 1.91-1.83 (m, 1H), 1.74-1.65 (m, 1H). MS *m*/*z* 485.4 M+H]⁺

### Example 2: Preparation of Compound 2

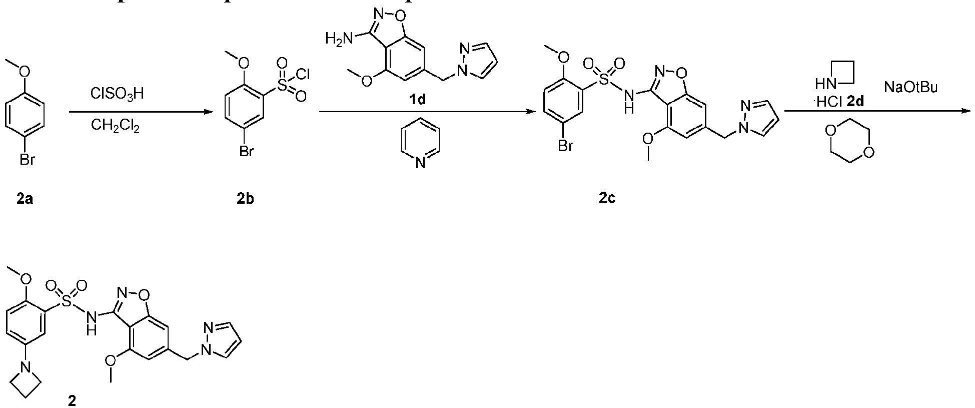

The P-Methoxybromobenzene **2a** (1.0 g, 5.38 mmol) was dissolved in dichloromethane (DCM, 5 mL). Then Chlorosulfonic acid (1.0 mL) was added under ice bath. The reaction was stirred at RT for 3 h. The reaction was poured into ice water and extracted with DCM (20 mL×3). The collected organic phase was washed with sat.NaCl, dried with anhydrous sodium sulfate, filterated and concentrated under reduced pressure. The crude was purified by column chromatography to give white solid compound **2b** (1.4 g, yield 92%).

The compound **Id** was synthesized according to WO2020254946. The compound **2b** (70 mg, 0.25 mmol) was dissolved in pyrdine (0.2 mL). Then **Id** (30 mg, 0.12 mmol) was added. The reaction mixture was stirred at 120 °C for 2 h in a sealed tube. The reaction was concentrated under reduced pressure. The crude was purified by column chromatography to give white solid compound **2c** (30 mg, yield 50%). MS *m*/*z* 493.3 [M+H]⁺

The compound **2c** (25 mg, 0.05 mmol), Methanesulfonic acid (2-dicyclohexylphosphine-2', 6'-diisopropyloxy-1,1 '- biphenyl) (2-amino-1,1'-biphenyl-2-yl) palladium (II) (4 mg, 0.005 mmol), 2-Dicyclohexylphosphate-20',6'-diisopropyloxy-1,1'- biphenyl (5 mg, 0.01 mmol), sodium tert-butoxide (19 mg, 0.20 mmol) and azetidine hydrochloride (14 mg, 0.15 mmol) were dissolved in dioxane (0.5 mL) The reaction mixture was stirred at 90 °C for 3 h in a sealed tube under N₂. TLC showed the reaction was completed. The mixture was filterated through diatomite and concentrated under reduced pressure. The crude was purified by Pre-TLC to give white solid compound 2 (3 mg, yield 13%). ¹H NMR (500 MHz, CDCl₃) δ 8.08 (s, 1H), 7.56 (d, *J* = 1.2 Hz, 1H), 7.43 (d, *J* = 2.0 Hz, 1H), 7.29 (br, 1H), 6.84 (d, *J* = 8.8 Hz, 1H), 6.78 (s, 1H), 6.73 (br, 1H), 6.44 (s, 1H), 6.31 (t, *J* = 1.9 Hz, 1H), 5.37 (s, 2H), 3.95 (s, 3H), 3.95-3.91 (m, 4H), 3.83 (s, 3H), 2.43-2.36 (m, 2H) ppm. MS *m*/*z* 470.4 [M+H]⁺.

### Example 3: Preparation of Compound 3

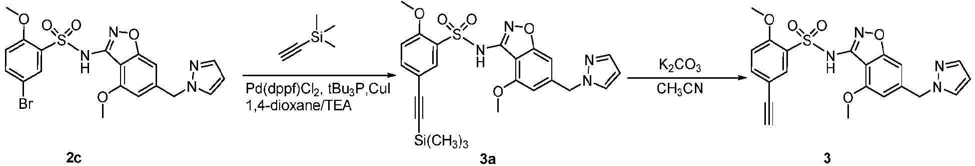

The compound **2c** (28 mg, 0.06 mmol), Trimethylsilyl acetylene (11 mg, 0.11 mmol), Pd(dppf)Cl₂ (9 mg, 0.01 mmol), Tri-tert-butylphosphine (6 mg, 0.03 mmol) and Cuprous iodide (CuI, 2 mg, 0.01 mmol) were dissolved in Tetrahydrofuran/triethylamine (0.5 mL/0.5 mL) The reaction mixture was stirred at 90 °C for 3 h in a sealed tube under N₂. The mixture was filterated through diatomite and concentrated under reduced pressure. The crude was purified by column chromatography to give white solid compound **3a** (9 mg, yield 31%). MS *m*/*z* 511.4 [M+H]⁺.

The compound **3a** (9 mg, 0.02 mmol) was dissolved in acetonitrile (0.1 mL). Then potassium carbonate (5 mg, 0.04 mmol) was added. The reaction mixture was stirred at RT for 2 h. The mixture was filterated through and concentrated under reduced pressure. The crude was purified by Pre-TLC to give white solid compound **3** (4 mg, yield 49%). ¹H NMR (500 MHz, CDCl₃) δ 8.27 (d, *J* = 2.1 Hz, 1H), 7.63 (dd, *J* = 8.6 Hz, *J* = 2.1 Hz, 1H), 7.57 (d, *J* = 1.6 Hz, 1H), 7.44 (d, *J* = 2.2 Hz, 1H), 6.90 (d, *J* = 8.6 Hz, 1H), 6.79 (s, 1H), 6.45 (s, 1H), 6.32 (t, *J* = 2.1 Hz, 1H), 5.38 (s, 2H), 3.96 (s, 3H), 3.92 (s, 3H), 3.07 (s, 1H) ppm. MS *m*/*z* 439.3 [M+H]⁺.

### Example 4: Preparation of Compound 4

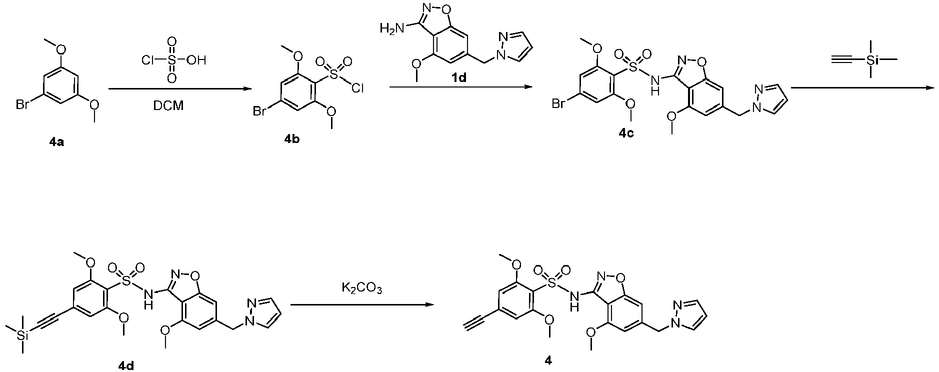

The compound **4a** (5.0 g, 23.03 mmol) was dissolved in dichloromethane (20 mL). Then Chlorosulfonic acid (3.0 mL) was added under ice bath. The reaction was stirred at RT for 1 h. The reaction was poured into ice water and extracted with DCM (20 mL×3). The collected organic phase was washed with sat.NaCl, dried with anhydrous sodium sulfate, filterated and concentrated under reduced pressure. The crude was purified by column chromatography to give white solid compound **4b** (500 mg). ¹HNMR (500 MHz, CDCl3) δ 6.82 (s, 2H), 3.98 (s, 6H)

The compound **4b** (150 mg, 0.48 mmol) was dissolved in pyrdine (0.2 mL). Then **Id** (58 mg, 0.24 mmol) was added. The reaction was stirred at 120 °C for 2 h in a sealed tube. The reaction was concentrated under reduced pressure. The crude was purified by column chromatography to give white solid compound **4c** (55 mg, yield 44%). MS *m*/*z* 523.0 [M+H]⁺.

The compound **4c** (35 mg, 0.07 mmol), triethylamine (0.2 mL), di (triphenylphosphine) palladium dichloride (5 mg, 0.007 mmol) and Trimethylsilyl acetylene (11 mg, 0.11 mmol) were dissolved in dimethyl sulfoxide (DMSO, 1 mL) The reaction was stirred at 100 °C for 2 h in a sealed tube under N₂. The mixture mixture was quenched with water and extracted with ethyl acetate (EA, 5 mL×3). The collected organic phase was washed with sat.NaCl, dried with anhydrous sodium sulfate, filterated and concentrated under reduced pressure. The crude was purified by column chromatography to give white solid compound **4d** (27 mg, yield 75%). MS *m*/*z* 541.2 [M+H]⁺.

The compound **4d** (27 mg, 0.05 mmol) was dissolved in MeOH (methanol, 0.2 mL) and N, N-dimethylformamide (DMF, 0.2 mL). Then potassium carbonate (35 mg, 0.25 mmol) was added. The reaction mixture was stirred at RT for 2 h. The mixture was filterated and concentrated under reduced pressure. The crude was purified by Pre-TLC to give white solid compound **3** (14 mg, yield 61%). MS m/z 469.0 [M+H]⁺. ¹H NMR (500 MHz, CDCl₃-*d*) δ 8.18 (s, 1H), 7.58 (d, *J* = 1.9 Hz, 1H), 7.45 (d, *J* = 2.3 Hz, 1H), 6.79 (s, 1H), 6.68 (s, 2H), 6.45 (s, 1H), 6.33 (t, *J* = 2.1 Hz, 1H), 5.39 (s, 2H), 3.96 (s, 3H), 3.87 (s, 6H), 3.21 (s, 1H) ppm.

### Example 5: Preparation of Compound 5

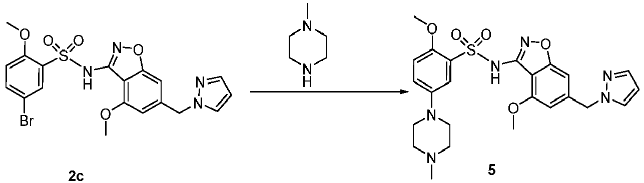

The compound **2c** (20 mg, 0.04 mmol), sodium tert-butoxide (12 mg, 0.12 mmol), Methanesulfonic acid (2-dicyclohexylphosphine-2', 6'-diisopropyloxy-1,1 '- biphenyl) (2-amino-1,1'-biphenyl-2-yl) palladium (II) (2.6 mg, 0.003 mmol), 2-Dicyclohexylphosphate-20',6'-diisopropyloxy-1,1'- biphenyl (2.6 mg, 0.006 mmol), and methylpiperazine (12 mg, 0.12 mmol) were dissolved in DMF (1 mL). The reaction mixture was stirred at 85 °C for 3 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC to give white solid compound 5 (8 mg, yield 38%). MS *m*/*z* 513.2 [M+H]⁺. ¹H NMR (500 MHz, DMSO-*d*₆) δ 9.90 (s, 1H), 7.86 (d, *J* = 2.1 Hz, 1H), 7.49 (d, *J* = 2.0 Hz, 1H), 7.39 (d, *J* = 3.0 Hz, 1H), 7.11 (dd, *J* = 9.0, 3.0 Hz, 1H), 7.00 (d, *J* = 9.0 Hz, 1H), 6.73 (s, 1H), 6.65 (s, 1H), 6.29 (t, *J* = 2.0 Hz, 1H), 5.41 (s, 2H), 3.83 (s, 3H), 3.68 (s, 3H), 3.09 (m, 4H), 2.74 (m, 4H), 2.42 (s, 3H).

### Example 6: Preparation of Compound 6

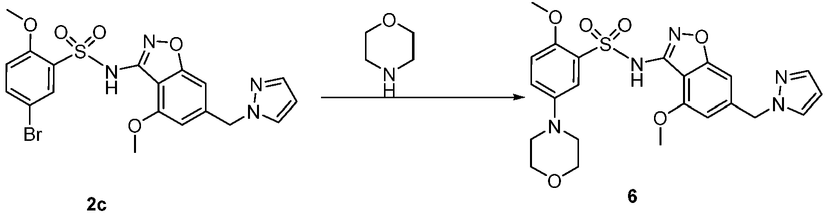

The compound **2c** (20 mg, 0.04 mmol), cesium carbonate (40 mg, 0.12 mmol), Methanesulfonic acid (2-dicyclohexylphosphine-2', 6'-diisopropyloxy-1,1 '- biphenyl) (2-amino-1,1'-biphenyl-2-yl) palladium (II) (2.6 mg, 0.003 mmol), 2-Dicyclohexylphosphate-20',6'-diisopropyloxy-1,1'- biphenyl (2.6 mg, 0.006 mmol), and Morpholine (11 mg, 0.12 mmol) were dissolved in DMF (1 mL). The reaction mixture was stirred at 100 °C for 3 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC to give white solid compound **6** (4 mg, yield 20%). MS m/z 500.1 [M+H]⁺. ¹H NMR (500 MHz, DMSO-*d*₆) δ 10.05 (s, 1H), 7.87 (d, *J* = 2.2 Hz, 1H), 7.49 (d, *J* = 2.2 Hz, 1H), 7.33 (d, *J* = 3.0 Hz, 1H), 7.15 (m, 2H), 6.78 (m, 2H), 6.30 (t, *J* = 2.0 Hz, 1H), 5.43 (s, 2H), 3.83 (s, 3H), 3.73-3.66 (m, 7H), 3.10-2.90 (m, 4H).

### Example 7: Preparation of Compound 7

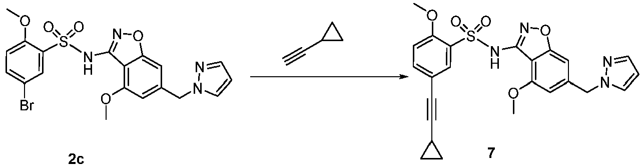

The compound **2c** (20 mg, 0.04 mmol), cesium carbonate (40 mg, 0.12 mmol), di (triphenylphosphine) palladium dichloride (2.8 mg, 0.004 mmol) and Cyclopropyl Acetylene (8 mg, 0.12 mmol) were dissolved in DMF (1 mL) The reaction mixture was stirred at 100 °C for 2 h in a sealed tube under N₂. The mixture was quenched with water and extracted with ethyl acetate (EA, 5 mL×3). The collected organic phase was washed with sat.NaCl, dried with anhydrous sodium sulfate, filterated and concentrated under reduced pressure. The crude was purified by column chromatography to give white solid compound 7 (5 mg, yield 26%). MS *m*/*z* 479.1 [M+H]⁺. ¹H NMR (500 MHz, DMSO-*d*₆) δ 10.46 (s, 1H), 7.88 (d, *J* = 2.0 Hz, 1H), 7.70 (d, *J* = 2.0 Hz, 1H), 7.64-7.58 (m, 1H), 7.50 (d, *J* = 1.3 Hz, 1H), 7.17 (d, *J* = 8.7 Hz, 1H), 6.85 (s, 1H), 6.75 (s, 1H), 6.31-6.29 (m, 1H), 5.44 (s, 2H), 3.81 (s, 3H), 3.78 (s, 3H), 1.54-1.49 (m, 1H), 0.89-0.83 (m, 2H), 0.73-0.75 (m, 2H).

### Example 8: Preparation of Compound 8

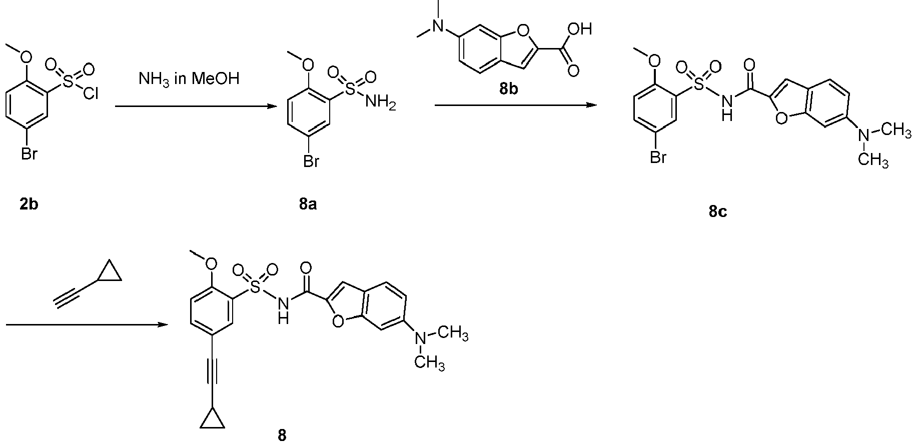

The compound **2b** (140 mg, 0.49 mmol) was dissolved in Tetrahydrofuran (5 mL). Then ammonia methanol solution (7 M, 1.0 mL, 7 mmol) was added. The reaction was stirred at RT for 1 h in a sealed tube. The reaction was concentrated under reduced pressure. The crude was purified by column chromatography to give white solid compound **8a** (100 mg, yield 77%).

The compound **8b** was synthesized according to WO2020216701. The compound **8a** (50 mg, 0.19 mmol), **8b** (47 mg, 0.21 mmol) and N,N-diisopropylethylamine (107 mg, 0.76 mmol) was dissolved in DCM (2 mL). Then 1H-Benzotriazol-1-yloxytripyrrolyl hexafluorophosphate (130 mg, 0.23 mmol) was added. The reaction was stirred at RT for overnight. The mixture was quenched with water and extracted with ethyl acetate (EA, 5 mL×3). The collected organic phase was washed with sat.NaCl (sat.: saturated), dried with anhydrous sodium sulfate, filterated and concentrated under reduced pressure. The crude was purified by column chromatography to give white solid compound **8c** (50 mg, yield 59%). MS *m*/*z* 453.0 [M+H]⁺

The compound **8c** (25 mg, 0.06 mmol), cesium carbonate (54 mg, 0.17 mmol), di (triphenylphosphine) palladium dichloride (3.9 mg, 0.006 mmol) and Cyclopropyl Acetylene (11 mg, 0.17 mmol) were dissolved in DMF (1 mL) The reaction mixture was stirred at 100 °C for 2 h in a sealed tube under N₂. The mixture was quenched with water and extracted with ethyl acetate (EA, 5 mL×3). The collected organic phase was washed with sat.NaCl, dried with anhydrous sodium sulfate, filterated and concentrated under reduced pressure. The crude was purified by Pre-TLC to give white solid compound **8** (11 mg, yield 46%). MS m/z 439.0 [M+H]⁺. ¹H NMR (500 MHz, DMSO-*d*₆) δ 12.55 (s, 1H), 7.91 (br, 1H), 7.80 (d, *J* = 2.1 Hz, 1H), 7.63 (s, 1H), 7.55 (d, *J* = 8.8 Hz, 1H), 7.18 (d, *J* = 7.8 Hz, 1H), 6.85 (d, *J =* 8.8 Hz, 1H), 6.77 (s, 1H), 3.86 (s, 3H), 2.98 (s, 6H), 1.58-1.52 (m, 1H), 0.90-0.87 (m, 2H), 0.80 - 0.74 (m, 2H).

### Example 9: Preparation of Compound 9

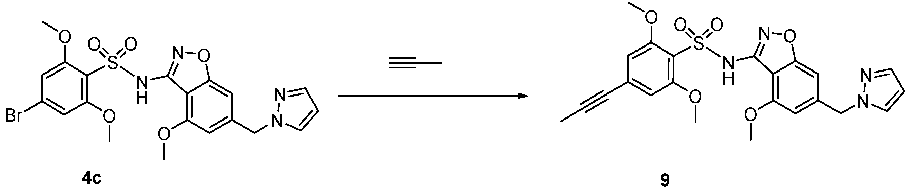

The compound **4c** (80 mg, 0.15 mmol), propyne (1 M, 0.45 mmol), cesium carbonate (149 mg, 0.46 mmol) and di (triphenylphosphine) palladium dichloride (14 mg, 0.02 mmol) were dissolved in DMF (1 mL) The reaction mixture was stirred at 100 °C for 3 h i under N₂. The mixture was filterated through diatomite and concentrated under reduced pressure. The crude was purified by Pre-TLC (MeOH: DCM = 1:20, V:V) to give compound **9** (7 mg, yield 9%). ¹H NMR (500 MHz, DMSO-*d*₆) δ 9.85 (s, 1H), 7.88 (d, *J* = 2.1 Hz, 1H), 7.50 (d, *J* = 1.4 Hz, 1H), 6.90-6.70 (m, 4H), 6.30 (t, *J* = 2.0 Hz, 1H), 5.44 (s, 2H), 3.84 (s, 3H), 3.74 (s, 6H), 2.07 (s, 3H) ppm. MS *m*/*z* 483.0 [M+H]⁺.

### Example 10: Preparation of Compound 10

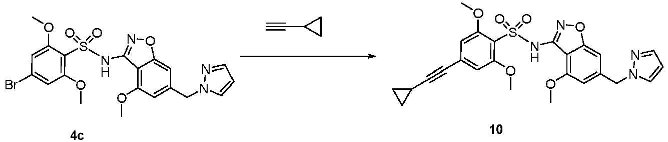

The compound **4c** (50 mg, 0.10 mmol), Cyclopropyl Acetylene (13 mg, 0.19 mmol), cesium carbonate (93 mg, 0.29 mmol) and di (triphenylphosphine) palladium dichloride (13 mg, 0.02 mmol) were dissolved in DMF (1 mL). The reaction mixture was stirred at 100 °C for 3 h under N₂. The mixture was filterated through diatomite and concentrated under reduced pressure. The crude product was purified by Pre-TLC (MeOH: DCM = 1:20, V:V) to give compound **10** (12 mg, yield 25%). ¹H NMR (500 MHz, DMSO-*d*₆) δ 9.79 (brs, 1H), 7.88 (d, *J* = 2.1 Hz, 1H), 7.50 (d, *J* = 1.4 Hz, 1H), 6.82 (s, 1H), 6.77-6.68 (m, 3H), 6.30 (t, *J* = 2.0 Hz, 1H), 5.44 (s, 2H), 3.85 (s, 3H), 3.74 (s, 6H), 1.60-1.53 (m, 1H), 0.95-0.89 (m, 2H), 0.80-0.73 (m, 2H) ppm. MS m/z 508.9 [M+H]⁺.

### Example 11: Preparation of Compound 11

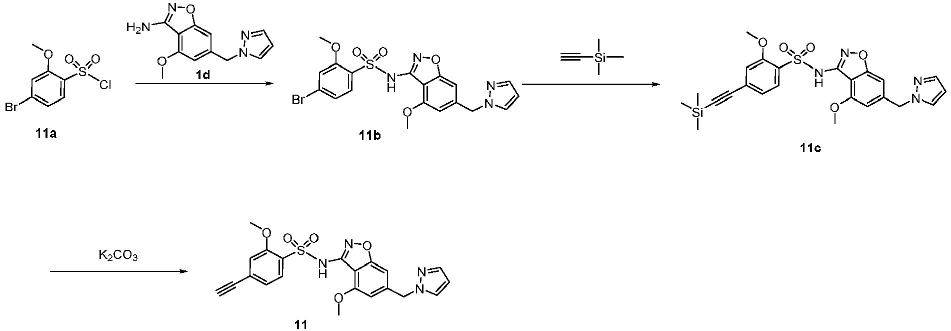

The compound **11a** was synthesized according to WO2006610629. The compound **Ila** (300 mg, 1.05 mmol) was dissolved in pyrdine (1.2 mL). Then **Id** (128 mg, 0.53 mmol) was added. The reaction mixture was stirred at 120 °C for 2 h in a sealed tube. The reaction mixture was concentrated under reduced pressure. The crude was purified by column chromatography to give white solid compound **11b** (100 mg, yield 39%). MS *m*/z 494.9 [M+H]⁺.

The compound **11b** (18 mg, 0.04 mmol), triethylamine (0.2 mL), di (triphenylphosphine) palladium dichloride (2 mg, 0.003 mmol) and Trimethylsilyl acetylene (12 mg, 0.12 mmol) were dissolved in dimethyl sulfoxide (1 mL) The reaction mixture was stirred at 100 °C for 2 h in a sealed tube under N₂. The mixture was quenched with water and extracted with ethyl acetate (EA, 5 mL×3). The collected organic phase was washed with sat.NaCl, dried with anhydrous sodium sulfate, filterated and concentrated under reduced pressure. The crude was purified by column chromatography to give white solid product **11c** (5 mg, yield 27%). MS *m*/*z* 511.1 [M+H]⁺.

The compound **11c** (5 mg, 0.01 mmol) was dissolved in MeOH (1 mL) and N,N-dimethylformamide (DMF, 0.2 mL). Then potassium carbonate (4 mg, 0.03 mmol) was added. The reaction was stirred at RT for 2 h. The mixture was filterated through and concentrated under reduced pressure. The crude was purified by Pre-TLC (MeOH: DCM = 1:20, V:V) to give white solid compound **11** (2 mg, yield 47%). ¹H NMR (500 MHz, CDCl₃) δ 8.08 (d, *J* = 8.1 Hz, 1H), 8.02 (s, 1H), 7.57 (d, *J* = 1.2 Hz, 1H), 7.43 (d, *J* = 2.0 Hz, 1H), 7.20 (d, *J* = 8.1 Hz, 1H), 7.03 (s, 1H), 6.79 (s, 1H), 6.45 (s, 1H), 6.32 (t, *J* = 2.0 Hz, 1H), 5.38 (s, 2H), 3.96 (s, 3H), 3.91 (s, 3H), 3.24 (s, 1H) ppm. MS m/z 439.0 [M+H]⁺.

### Example 12: Preparation of Compound 12

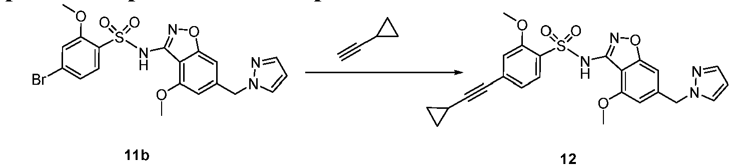

The compound **11b** (18 mg, 0.04 mmol), cesium carbonate (40 mg, 0.12 mmol), di (triphenylphosphine) palladium dichloride (2.8 mg, 0.004 mmol) and Cyclopropyl Acetylene (8 mg, 0.12 mmol) were dissolved in DMF (1 mL). The reaction mixture was stirred at 100 °C for 2 h under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC (MeOH: DCM = 1:20, V:V) to give white solid compound **12** (10 mg, yield 57%). ¹H NMR (500 MHz, DMSO-*d*₆) δ 10.34 (s, 1H), 7.87 (d, *J* = 2.1 Hz, 1H), 7.71 (d, *J* = 8.1 Hz, 1H), 7.50 (d, *J* = 1.6 Hz,1H), 7.13 (s, 1H), 7.05 (d, *J* = 8.1 Hz, 1H), 6.83 (s, 1H), 6.73 (s, 1H), 6.30 (t, *J* = 2.1 Hz, 1H), 5.44 (s, 2H), 3.81 (s, 3H), 3.76 (s, 3H), 1.58 (m, 1H), 0.97-0.87 (m, 2H), 0.80-0.70 (m, 2H) ppm. MS *m*/*z* 479.1 [M+H]⁺.

### Example 13: Preparation of Compound 13

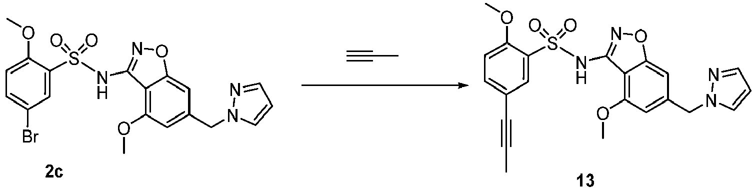

The compound **2c** (30 mg, 0.06 mmol), propyne in THF (1 M, 0.30 mmol), cesium carbonate (60 mg, 0.18 mmol) and di (triphenylphosphine) palladium dichloride (8 mg, 0.01 mmol) were dissolved in DMF (1 mL) The reaction was stirred at 100 °C for 3 h in a sealed tube under N₂. The reaction mixture was filterated through diatomite and concentrated under reduced pressure. The crude was purified by Pre-TLC (MeOH: DCM = 1:20, V:V) to give compound **13** (1.6 mg, yield 6%). ¹H NMR (500 MHz, DMSO-*d*₆) δ 10.50 (s, 1H), 7.87 (d, *J =* 1.6 Hz, 1H), 7.73 (br, 1H), 7.62 (br, 1H), 7.49 (s, 1H), 7.18 (br, 1H), 6.84 (br, 1H), 6.74 (br, 1H), 6.30 (s, 1H), 5.44 (s, 2H), 3.81 (s, 3H), 3.78 (s, 3H), 2.02 (s, 3H). MS *m*/*z* 453.0 [M+H]⁺.

### Example 14: Preparation of Compound 14

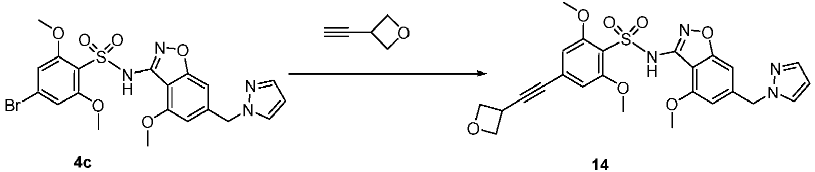

The compound **4c** (60 mg, 0.11 mmol), 3-ethynyloxycyclohexane (19 mg, 0.23 mmol), cesium carbonate (112 mg, 0.34 mmol) and di (triphenylphosphine) palladium dichloride (13 mg, 0.12 mmol) were dissolved in DMF (1 mL) The reaction mixture was stirred at 100 °C for 2 h in a sealed tube under N₂. The mixture was filterated through diatomite and concentrated under reduced pressure. The crude was purified by Pre-TLC (MeOH: DCM = 1:20) to give compound **14** (13 mg, yield 22%). ¹H NMR (500 MHz, DMSO-*d*₆) δ 9.88 (s, 1H), 7.88 (d, *J =* 2.0 Hz, 1H), 7.50 (d, *J* = 1.4 Hz, 1H), 6.86-6.80 (m, 3H), 6.76 (s, 1H), 6.30 (t, *J* = 2.0 Hz, 1H), 5.45 (s, 2H), 4.80 (dd, *J* = 8.5, 5.5 Hz, 2H), 4.62 (dd, *J* = 7.0, 5.5 Hz, 2H), 4.22-4.14 (m, 1H), 3.85 (s, 3H), 3.77 (s, 6H) ppm. MS *m*/*z* 525.0 [M+H]⁺.

### Example 15: Preparation of Compound 15

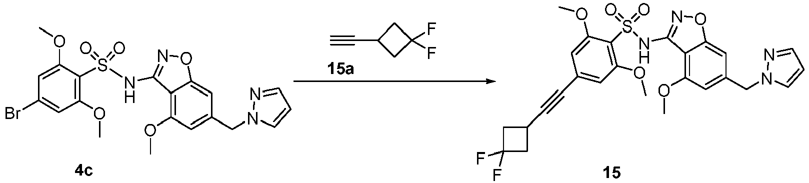

The compound **4c** (60 mg, 0.11 mmol), **15a** (40 mg, 0.34 mmol), cesium carbonate (112 mg, 0.34 mmol) and di(triphenylphosphine) palladium dichloride (16 mg, 0.02 mmol) were dissolved in DMF (1 mL). The reaction mixture was stirred at 100 °C for 2 h under N₂. The mixture was filterated through diatomite and concentrated under reduced pressure. The crude was purified by Pre-TLC (MeOH: DCM = 1:20, V:V) to give compound **15** (14 mg, yield 22%). ¹H NMR (500 MHz, DMSO-*d*₆) δ 9.90 (s, 1H), 7.89 (d, *J* = 2.1 Hz, 1H), 7.50 (d, *J* = 1.3 Hz, 1H), 6.83 (s, 1H), 6.80 (s, 2H), 6.76 (s, 1H), 6.31 (t, *J* = 2.0 Hz, 1H), 5.45 (s, 2H), 3.85 (s, 3H), 3.76 (s, 6H), 3.32-3.25 (m, 1H), 3.10-3.00 (m, 2H), 2.81-2.69 (m, 2H) ppm. MS *m*/*z* 559.0 [M+H]⁺.

### Example 16: Preparation of Compound 16

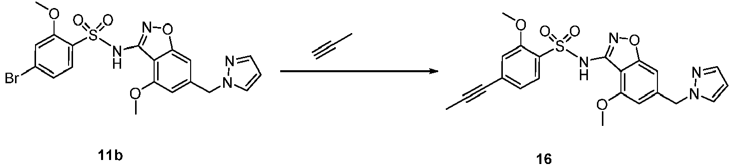

The compound **11b** (28 mg, 0.06 mmol), cesium carbonate (59 mg, 0.18 mmol) and di (triphenylphosphine) palladium dichloride (2 mg, 0.003 mmol) and propyne (24 mg, 0.6 mmol) were dissolved in DMF (1 mL) The reaction mixture was stirred at 100 °C for 3 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC (MeOH: DCM = 1:20, V:V) to give white solid compound **16** (22 mg, yield 86%). ¹H NMR (500 MHz, CDCl₃) δ 8.06-7.97 (m, 2H), 7.57 (d, *J* = 1.6 Hz, 1H), 7.43 (d, *J* = 2.1 Hz, 1H), 7.08 (dd, *J* = 8.1, 1.2 Hz, 1H), 6.92 (d, *J* = 1.0 Hz, 1H), 6.79 (s, 1H), 6.44 (s, 1H), 6.32 (t, *J* = 2.1 Hz, 1H), 5.38 (s, 2H), 3.96 (s, 3H), 3.88 (s, 3H), 2.05 (s, 3H) ppm. MS *m*/*z* 453.0 [M+H]⁺.

### Example 17: Preparation of Compound 17

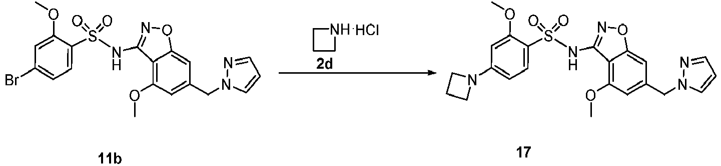

The compound **11b** (20 mg, 0.04 mmol), sodium tert-butoxide (31 mg, 0.32 mmol), Methanesulfonic acid (2-dicyclohexylphosphine-2', 6'-diisopropyloxy-1,1 '- biphenyl) (2-amino-1,1'-biphenyl-2-yl) palladium (II) (2.6 mg, 0.006 mmol), 2-Dicyclohexylphosphate-20',6'-diisopropyloxy-1,1'- biphenyl (5 mg, 0.01 mmol) and **2d** (19 mg, 0.2 mmol) was dissolved in DMF (1 mL). The reaction mixture was stirred at 90 °C for 2 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC (MeOH: DCM = 1:20, V:V) to give white solid compound **17** (8 mg, yield 42%). ¹H NMR (500 MHz, CDCl₃) δ 7.97 (s, 1H), 7.88 (d, *J* = 8.7 Hz, 1H), 7.61 (s, 1H), 7.50 (s, 1H), 6.77 (s, 1H), 6.50 (s, 1H), 6.36 (s, 1H), 5.97 (dd, *J* = 8.7, 1.9 Hz, 1H), 5.71 (d, *J =* 1.9 Hz, 1H), 5.42 (s, 2H), 3.99-3.92 (m, 7H), 3.83 (s, 3H), 2.47-2.35 (m, 2H) ppm. MS *m*/*z* 470.0 [M+H]⁺.

### Example 18: Preparation of Compound 18

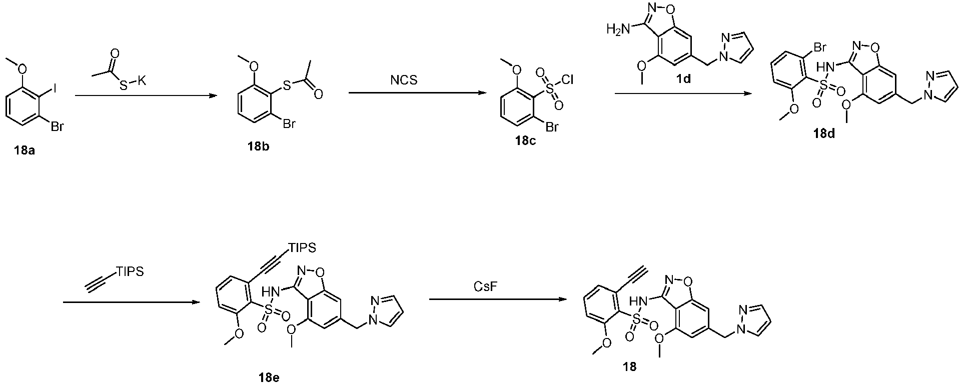

The compound **18a** (1.6 g, 5.11 mmol), potassium thioacetate (875 mg, 7.66 mmol), 1,10-Phenanthroline (460 mg, 2.56 mmol), and cuprous iodide (460 mg, 2.56 mmol) was dissolved in toluene (15 mL). The reaction mixture was stirred at 100 °C for 20 h. The mixture was quenched with water and extracted with ethyl acetate (20 mL×3). The collected organic phase was washed with sat.NaCl, dried with anhydrous sodium sulfate, filterated and concentrated under reduced pressure. The crude was purified by column chromatography to give reddish brown solid compound **18b** (1.0 g, yield 75%).

The N-chlorosuccinimide (2.3 g, 17.2 mmol) was dissolved in hydrochloric acid aqueous solution (2 M, 2 mL) and MeCN (10 mL). Then **18b** (1.1 g, 4.2 mmol) in MeCN (2 mL) was added dropwise at RT. The reaction mixture was stirred at RT for 1 h. The mixture was quenched with water and extracted with ethyl acetate (20 mL×3). The collected organic phase was washed with sat.NaCl, dried with anhydrous sodium sulfate, filterated and concentrated under reduced pressure. The crude was purified by column chromatography to give reddish brown solid compound **18c** (1.0 g, yield 83%). ¹H NMR (500 MHz, CDCl₃) δ 7.47 - 7.39 (m, 2H), 7.10 (dd, J = 7.5, 2.1 Hz, 1H), 4.05 (s, 3H).

The compound **18c** (117 mg, 0.41 mmol) was dissolved in pyrdine (0.2 mL). Then Id (50 mg, 0.20 mmol) was added. The reaction mixture was stirred at 120 °C for 1 h in a sealed tube. The reaction was concentrated under reduced pressure. The crude was purified by column chromatography to give white solid compound **18d** (50 mg, yield 50%). MS *m*/*z* 492.8 [M+H]⁺.

The compound **18d** (25 mg, 0.05 mmol), triethylamine (0.2 mL), di (triphenylphosphine) palladium dichloride (5 mg, 0.007 mmol) and Triisopropylsilyacetylene (27 mg, 0.15 mmol) were dissolved in dimethyl sulfoxide (1 mL) The reaction was stirred at 100 °C for overnight in a sealed tube under N₂. The mixture was quenched with water and extracted with ethyl acetate (EA, 5 mL×3). The collected organic phase was washed with sat.NaCl, dried with anhydrous sodium sulfate, filterated and concentrated under reduced pressure. The crude was purified by column chromatography to give white solid compound **18e** (25 mg, yield 83%). MS *m*/*z* 595.1 [M+H]⁺.

The compound **18e** (10 mg, 0.017 mmol) was dissolved in DMF (1 mL). Then Cesium Fluoride (15 mg, 0.1 mmol) was added. The reaction mixture was stirred at RT for 5 h. The mixture was filterated and concentrated under reduced pressure. The crude was purified by Pre-TLC (MeOH: DCM = 1:20) to give white solid compound **18** (3 mg, yield 40%). MS *m*/*z* 439.0 [M+H]⁺. ¹H NMR (500 MHz, CDCl₃) δ 8.18 (s, 1H), 7.67 (s, 1H), 7.53 (s, 1H), 7.42 (t, *J* = 8.1 Hz, 1H), 7.29 (d, *J* = 7.7 Hz, 1H), 6.96 (d, *J* = 8.4 Hz, 1H), 6.83 (s, 1H), 6.66 (s, 1H), 6.40 (s, 1H), 5.49 (s, 2H), 3.99 (s, 3H), 3.91 (s, 3H), 3.58 (s, 1H).

### Example 19: Preparation of Compound 19

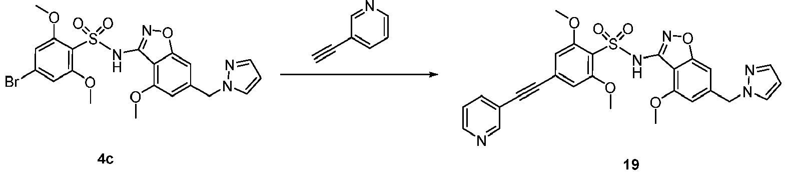

The compound **4c** (25 mg, 0.05 mmol), cesium carbonate (46 mg, 0.14 mmol) and di (triphenylphosphine) palladium dichloride (2.8 mg, 0.004 mmol) and 3-alkynylpyridine (25 mg, 0.24 mmol) were dissolved in DMF (1 mL) The reaction mixture was stirred at 100 °C for 2 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude product was purified by Pre-TLC (MeOH: DCM = 1:20, V:V) to give white solid compound **19** (12 mg, yield 46%). MS m/z 546.1 [M+H]⁺.

### Example 20: Preparation of Compound 19'

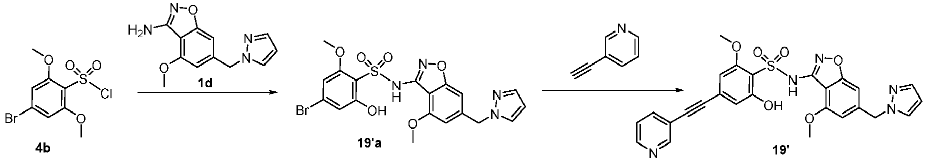

The compound **4b** (150 mg, 0.48 mmol) was dissolved in pyrdine (0.2 mL). Then **Id** (58 mg, 0.24 mmol) was added. The reaction was stirred at 120 °C for 1 h in a sealed tube. The reaction was concentrated. The crude was purified by column chromatography to give white solid compound **19'a** (10 mg, yield 8%). MS *m*/*z* 508.9 [M+H]⁺.

The compound **19'a** (10 mg, 0.02 mmol), cesium carbonate (19 mg, 0.06 mmol) and di (triphenylphosphine) palladium dichloride (1.4 mg, 0.002 mmol) and 3-alkynylpyridine (10 mg, 0.10 mmol) were dissolved in DMF (1 mL) The reaction mixture was stirred at 100 °C for 2 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC to give white solid compound **19'** (2 mg, yield 19%). ¹H NMR (500 MHz, DMSO-*d*₆) δ 14.01 (s, 1H), 8.77 (d, *J* = 1.1 Hz, 1H), 8.60 (dd, *J* = 4.8, 1.3 Hz, 1H), 8.02 - 7.97 (m, 1H), 7.86 (d, *J* = 1.6 Hz, 1H), 7.51 - 7.44 (m, 2H), 6.69 - 6.52 (m, 4H), 6.30 (t, *J* = 1.9 Hz, 1H), 5.41 (s, 2H), 3.83 (s, 3H), 3.78 (s, 3H). MS *m*/*z* 532.0 [M+H]⁺.

### Example 21: Preparation of Compound 20

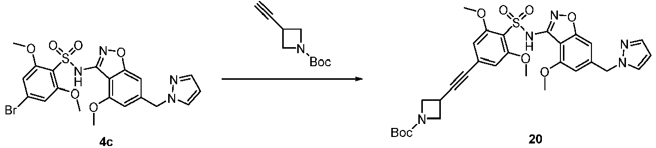

The compound **4c** (30 mg, 0.06 mmol), cesium carbonate (60 mg, 0.18 mmol), di (triphenylphosphine) palladium dichloride (2.8 mg, 0.003 mmol) and 3-ethynyl-1-azacyclobutanoic acid tert butyl ester (41 mg, 0.23 mmol) were dissolved in DMF (1 mL) The reaction mixture was stirred at 100 °C for 2 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC to give white solid compound **20** (25 mg, yield 70%). ¹H NMR (500 MHz, DMSO-*d*₆) δ 9.81 (s, 1H), 7.87 (d, *J* = 1.6 Hz, 1H), 7.50 (d, *J* = 1.0 Hz, 1H), 6.85 - 6.80 (m, 3H), 6.75 (s, 1H), 6.34 - 6.24 (m, 1H), 5.45 (s, 2H), 4.21 - 4.12 (m, 2H), 3.90 - 3.81 (m, 5H), 3.77 (s, 6H), 3.73 - 3.62 (m, 1H), 1.38 (s, 9H). MS m/z 624.1 [M+H]⁺.

### Example 22: Preparation of Compound 21

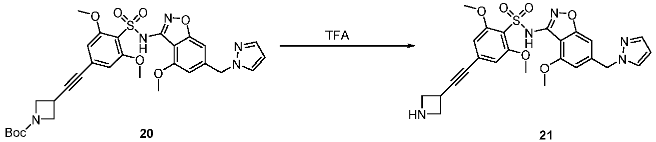

The compound **20** (7 mg, 0.01 mmol) was dissolved in DCM (1 mL). Then trifluoroacetic acid (0.3 mL) was added. The reaction was stirred at RT for 2 h. The mixture was concentrated under reduced pressure to afford compound **21** (6.9 mg, yield 100%). ¹H NMR (500 MHz, DMSO-*d*₆) δ 9.92 (s, 1H), 8.86 (s, 2H), 7.88 (d, *J* = 1.7 Hz, 1H), 7.50 (d, *J* = 1.1 Hz, 1H), 6.86 - 6.72 (m, 4H), 6.30 (t, *J* = 1.9 Hz, 1H), 5.45 (s, 2H), 4.31 - 4.15 (m, 2H), 4.11 - 3.92 (m, 3H), 3.86 (s, 3H), 3.77 (s, 6H). MS m/z 524.1 [M+H]⁺.

### Example 23: Preparation of Compound 22

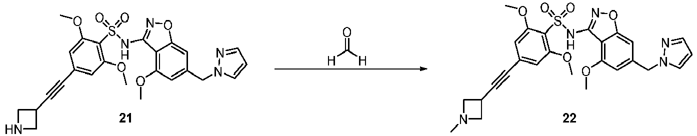

The compound **21** trifluoroacetate salt (20 mg, 0.03 mmol) was dissolved in DCM (1 mL). Then formaldehyde solution (0.1 mL, 37%) was added. The reaction mixture was stirred at RT for 1 h and Sodium triacetoxyborohydride (40 mg, 0.19 mmol) was added. The reaction was stirred at RT for another 1 h. The mixture was concentrated under reduced pressure. The crude was purified by Pre-TLC (MeOH: DCM = 1:10) to give white solid compound **22** (3 mg, yield 17%). ¹H NMR (500 MHz, DMSO-*d*₆) δ 9.82 (s, 1H), 7.87 (s, 1H), 7.50 (s, 1H), 6.92 - 6.62 (m, 4H), 6.38 - 6.19 (m, 1H), 5.44 (s, 2H), 3.85 (s, 3H), 3.74 (s, 6H), 3.64 - 3.57 (m, 2H), 3.48 - 3.40 (m, 1H), 3.17 - 3.09 (m, 2H), 2.27 (s, 3H). MS m/z 538.0 [M+H]⁺.

### Example 24: Preparation of Compound 23

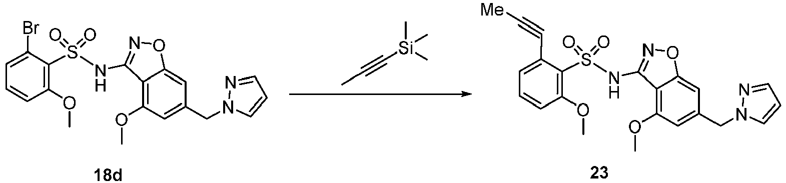

The compound **18d** (40 mg, 0.08 mmol), cesium carbonate (80 mg, 0.24 mmol) and di (triphenylphosphine) palladium dichloride (2.8 mg, 0.004 mmol) and 1-(Trimethylsilyl) propargyl (45 mg, 0.4 mmol) were dissolved in DMF ( 1 mL) The reaction mixture was stirred at 100 °C for 2 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC (MeOH: DCM = 1:20) to give white solid compound **23** (3 mg, yield 8%). ¹H NMR (500 MHz, CDCl₃) δ 8.18 (s, 1H), 7.64 (s, 1H), 7.50 (s, 1H), 7.35 (t, *J* = 8.1 Hz, 1H), 7.16 (d, *J* = 7.8 Hz, 1H), 6.91 - 6.75 (m, 2H), 6.59 (s, 1H), 6.38 (s, 1H), 5.45 (s, 2H), 3.98 (s, 3H), 3.87 (s,3H), 2.13 (s, 3H).MS m/z 452.9 [M+H]⁺.

### Example 25: Preparation of Compound 24

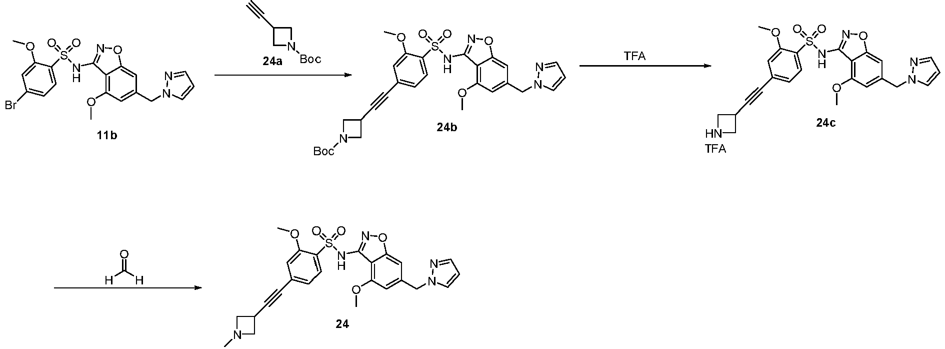

The compound **11b** (25 mg, 0.05 mmol), cesium carbonate (49 mg, 0.15 mmol) and di (triphenylphosphine) palladium dichloride (2.0 mg, 0.003 mmol) and 3-ethynyl-1-azacyclobutanoic acid tert butyl ester **24a** (28 mg, 0.15 mmol) were dissolved in DMF ( 1 mL) The reaction mixture was stirred at 100 °C for 2 h in a sealed tube under N₂. The mixture was quenched with water and extracted with ethyl acetate (5 mL×3). The collected organic phase was washed with sat.NaCl, dried with anhydrous sodium sulfate, filterated and concentrated under reduced pressure. The crude was purified by Pre-TLC to give white solid compound **24b** (20 mg, yield 67%). MS m/z 594.0 [M+H]⁺.

The compound **24b** (20 mg, 0.03 mmol) was dissolved in DCM (1 mL). Then trifluoroacetic acid (0.3 mL) was added. The reaction was stirred at RT for 2 h. The mixture was concentrated under reduced pressure to afford compound **24c** (20 mg, yield 100%). MS m/z 494.0 [M+H]⁺.

The compound **24c** (20 mg, 0.03 mmol) was dissolved in DCM (1 mL). Then formaldehyde solution (0.1 mL, 37%) was added. The reaction was stirred at RT for 1 h and Sodium triacetoxyborohydride (40 mg, 0.19 mmol) was added. The reaction was stirred at RT for another 1 h. The mixture was concentrated under reduced pressure. The crude was purified by Pre-TLC (MeOH: DCM = 1:10) to give white solid compound **24** (3 mg, yield 18%). ¹H NMR (500 MHz, DMSO) δ 10.19 (br, 1H), 7.85 (d, *J* = 2.2 Hz, 1H), 7.74 (d, *J* = 8.0 Hz, 1H), 7.50 - 7.46 (m, 1H), 7.08 - 7.04 (m, 1H), 7.01 (dd, *J* = 8.0, 1.3 Hz, 1H), 6.66 (s, 1H), 6.60 (s, 1H), 6.28 (t, *J* = 2.0 Hz, 1H), 5.39 (s, 2H), 3.92 (t, *J* = 7.6 Hz, 2H), 3.81 (s, 3H), 3.74 (s, 3H), 3.69 - 3.61 (m, 1H), 3.61 - 3.52 (m, 2H), 2.52 (s, 3H). MS m/z 508.0 [M+H]⁺.

### Example 26: Inhibition of KAT6A enzyme activity by compounds

1x experimental buffer (modified Tris buffer) was prepared, and the compounds were dissolved with 100% DMSO to form 10 mM reservoir solution and diluted according to a certain concentration gradient. The compounds were transferred to a 384-well plate using an automatic sampler Echo, and the final concentration of DMSO was 1%. 1x KAT6A enzyme (catalytic domain) solution was prepared. A mixed solution of [3H]-acetyl-CoA (PERKIN ELMER, Cat. No. NET290250UC) and the substrate peptide H3 (1-21) was prepared. 10 µL of the enzyme solution was transferred to the 384-well plate and incubated at room temperature for 15 minutes. 10 µL of the mixed solution of [3H]-acetyl-CoA and the substrate peptide H3 (1-21) were added to the plate to initiate the reaction. The plate was incubated at room temperature for 1 hour. 10 µL of the stop solution was added to terminate the reaction. 25 µL of the reaction solution was transferred to a Flashplate (Perkin Elmer, Cat. No. SMP410A001PK) and incubated at room temperature for 1 hour. The plate was read using Microbeta. The inhibition rate of the compounds was calculated using the formula of Excel: inhibition% = (Max-Signal) / (Max - Min)*100%. Where max is the value of the DMSO control, min is the value of the non-enzymatic control, and signal is the value of the well where the compound was tested. The curve was fitted using XLFit software and IC₅₀ was calculated. The activities of some representative compounds are shown in Table 1.

**Table 1 KAT6A Enzymatic Activity of Compounds (IC₅₀, nM)**

| Compounds | KAT6A IC₅₀ (nM) |
|---|---|
| **1** | < 5 |
| **2** | < 10 |
| **3** | < 20 |
| **4** | < 5 |
| **5** | < 200 |
| **6** | < 100 |
| **7** | < 10 |
| **8** | < 1000 |
| **9** | < 10 |
| **10** | < 5 |
| **11** | < 10 |
| **12** | < 10 |
| **13** | < 20 |
| **14** | 67%* |
| **15** | < 5 |
| **16** | < 10 |
| **17** | 75%* |
| **19** | < 5 |
| **19'** | < 10 |
| **20** | < 5 |
| **21** | < 10 |
| **22** | < 20 |
| **23** | < 20 |
| **24** | < 20 |

| | |
|---|---|
| Inhibition% of compounds at the concentration of 11 nM | |

### Example 27: Pharmacokinetic study in rats

Instruments: XEVO TQ-S LC/MS instrument was produced by Waters. All measurement data were collected and processed by Masslynx V4.1 software, and the data were calculated and processed by Microsoft Excel. The statistical moment method of WinNonLin 8.0 software was used to calculate the pharmacokinetic parameters. These mainly include kinetic parameters Tmax, T1/2, Cmax, AUC₀₋₂₄ₕ etc. Column: ACQUITY UPLC BEH C18 (2.1 mm × 50 mm, 1.7 µm); column temperature 40°C; mobile phase A is water (0.1% formic acid), mobile phase B is acetonitrile, flow rate is 0.350 mL/min, gradient elution is adopted, the elution gradient is 0.50 min: 10% B; 1.50 min: 90% B; 2.50 min: 90% B; 2.51 min: 10% B; 3.50 min: stop. Injection volume: 1 µL.

Animals: 3 male SD rats with a weight range of 200-220 g were purchased and kept in the laboratory of the Experimental Animal Center for 2 days and then used. They were fasted for 12 hours predose and 4 hours after dosing. Drinking water was free during the test. After the rats were gavage, blood samples were taken according to the established time point.

Solvent: 0.4% ethanol + 0.4% Tween80 + 99.2% (0.5% methylcellulose M450). Preparation of the solution for intragastrical administration: the compounds were accurately weighed and added to the solvent, and ultrasound was used at room temperature for 5 minutes to completely dissolve the drug, and a 0.3 mg/mL medicinal solution was prepared.

Pharmaceutical samples: Generally, multiple samples with similar structures (with a molecular weight difference of more than 2 units) are taken, accurately weighed, and administered together (cassette PK). In this way, multiple compounds can be screened at the same time and their oral absorption rates can be compared. A single administration was also used to study the pharmacokinetics of the drug sample in rats.

After intragastrical administration, blood was taken from the orbit at 0.25, 0.5, 1, 2, 4, 8, 10 and 24 hours. Then 50 µL of the plasma sample was taken and added 200 µL of acetonitrile (including internal standard control verapamil 2 ng/mL), vortexed for 3 min and centrifuged at 20000 rcf, 4°C for 10 min. The supernatant plasma was used for LC-MS/ MS analysis.

The compounds were accurately weighed to prepare different concentrations, and quantitative analysis on mass spectrometry was performed to establish a standard curve, and then the concentration of the above-mentioned compound in the plasma was tested to obtain its concentration at different time points. All measurement data were collected and processed by relevant software, and the statistical moment method was used to calculate the pharmacokinetic parameters (mainly including kinetic parameters Tmax, T_{1/2}, Cmax, AUC₀₋₂₄ₕ etc). The kinetic parameters of some representative compounds are shown in Table 2.

**Table 2: Pharmacokinetic Parameters of Compounds in rats**

| Compound s | Dosage (mg/Kg) | T_{1/2} (h) | Tₘₐₓ (h) | Cₘₐₓ (ng/mL) | AUC₀₋₂₄ₕ (h*ng/mL) |
|---|---|---|---|---|---|
| **1** | 1.5 | 2.66 | 3.00 | 392.76 | 4481.07 |
| **4** | 3 | 8.04 | 9.33 | 6737.13 | 109958.71 |
| **11** | 2 | 5.29 | 1.33 | 6382.98 | 53744.39 |
| **12** | 3 | 24.59 | 2.00 | 4825.5 | 82170.11 |
| **14** | 3 | 6.62 | 3.33 | 427.98 | 5955.41 |
| **15** | 3 | 7.85 | 3.33 | 2124.12 | 32464.43 |
| **16** | 3 | 14.39 | 2.67 | 2794.27 | 45347.47 |
| **17** | 3 | 3.17 | 2.67 | 228.59 | 3117.46 |

All references mentioned in the present invention are cited as references in this application, just as each reference is cited separately. In addition, it should be understood that after reading the above lecture content of the present invention, those skilled in the art can make various modifications or modifications to the present invention, and these equivalent forms also fall within the scope of the claims attached to the present application.

## Claims

1. A compound of the following formula (I), or the optical isomers, pharmaceutically acceptable salts, prodrugs, deuterated derivatives, hydrates, or solvates thereof: 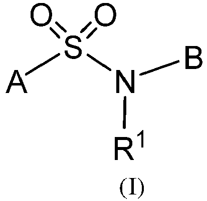 wherein in the formula (I):
A is selected from Formula (Ia), Formula (Ib), Formula (Ic) and Formula (Id): 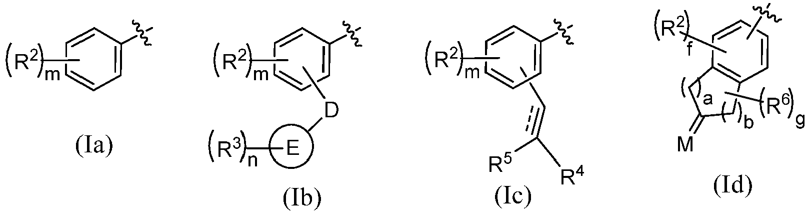
wherein, " 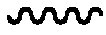 " refers to the site of formula (Ia) which attach to the remaining fragment of the compound of formula (I);
" 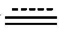 " represents a double or triple bond; and R⁵ is absent when it is a triple bond;
B is selected from Formula (Ie), Formula (If) and Formula (Ig): 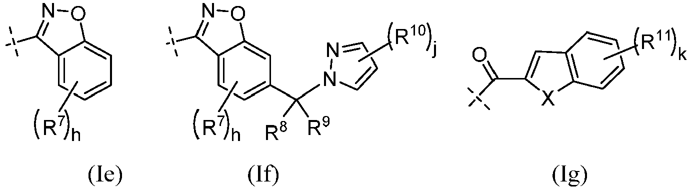
wherein, the "---" refers to the site of the fragment B which attach to the remaining fragment of the compound of formula (XIa);
R¹ is selected from the group consisting of hydrogen and C₁₋₄ alkyl;
each R² is independently selected from the group consisting of hydrogen, halogen, C₁₋₄ alkyl, C₁₋₄ haloalkyl, C₁₋₄ alkoxy-C₁₋₄ alkyl, C₁₋₄ haloalkoxy-C₁₋₄ alkyl, C₂₋₄ alkenyl, C₂₋₄ haloalkenyl, C₂₋₄ alkynyl, C₃₋₆ cycloalkyl, 3-to 8-membered heterocyclyl, aryl, heteroaryl, CN, OR^{a}, SR^{a}, and NR^{a}R^{a}; wherein, each R^{a} is independently selected from hydrogen, C₁₋₄ alkyl, C₁₋₄ haloalkyl, C₃₋₆ cycloalkyl, and 3-to 8-membered heterocyclyl;
D is selected from chemical bond, -O-, -S-, and-NR^{e}-; wherein R^{e} is selected from hydrogen, C₁₋₄ alkyl, and C₃₋₆ cycloalkyl; E is selected from C₃₋₈ cycloalkyl, 3-to 10-membered heterocyclyl, C₃₋₈ cycloalkyl-C₁₋₄ alkyl, and 3-to 10-membered heterocyclyl-C₁₋₄ alkyl;
each R³ is independently selected from hydrogen, halogen, C₁₋₄ alkyl, C₁₋₄ haloalkyl, CN, OR^{b}, SR^{b}, NR^{b}R^{b}, -C(O)R^{g}, -S(O)₂R^{g}, C₃₋₆ cycloalkyl, and 3-to 8-membered heterocyclyl; or two R³ which attach to a same carbon atom of the cycloalkyl or heterocyclyl ring form C=T together with the carbon atom; wherein, T is selected from O or CR¹²R¹³; wherein, R¹² and R¹³ are each independently selected from hydrogen, fluorine, or C₁₋₄ alkyl; the alkyl on the R¹² or R¹³ is optionally substituted with one or more groups selected from the group consisting: halogen, C₁₋₄ haloalkyl, CN, OR^{b}, SR^{b}, NR^{b}R^{b}, C₃₋₆ cycloalkyl, 3-to 8-membered heterocyclyl, aryl, and heteroaryl; each R^{b} is independently selected from hydrogen, C₁₋₄ alkyl, and C₁₋₄ haloalkyl; R^{g} is selected from hydrogen, C₁₋₄ alkyl, C₂₋₄ alkenyl, C₂₋₄ alkynyl, C₃₋₈ cycloalkyl, 3-to 8-membered heterocyclyl, aryl, and heteroaryl;
R⁴ and R⁵ are each independently selected from the group consisting of hydrogen, C₁₋₄ alkyl, C₃₋₈ cycloalkyl, 3-to 9-membered heterocyclyl, aryl, and heteroaryl; wherein the alkyl, cycloalkyl, heterocyclyl, aryl, or heteroaryl is optionally substituted with one or more groups selected from the group consisting of halogen, CN, OR^{a}, SR^{a}, NR^{a}R^{a}, NO₂, -C(O)R^{m}, -C(O)OR^{a}, -S(O)₂R^{m}, -C(O)NR^{a}R^{a}, -OC(O)R^{m}, -NR^{a}C(O)R^{m}, -NR^{a}S(O)₂R^{m}, C₁₋₄ alkyl, C₂₋₄ alkenyl, C₂₋₄ alkynyl, C₃₋₆ cycloalkyl, 3-to 8- membered heterocyclyl, aryl, and heteroaryl; or the cycloalkyl or heterocyclic group is substituted by =T; wherein, the definition of each R^{a} is as described as above; R^{m} is selected from C₁₋₄ alkyl, C₂₋₄ alkenyl, C₂₋₄ alkynyl, and C₃₋₆ cycloalkyl; the definition of T is described as above;
each R⁶ is independently selected from hydrogen, halogen, C₁₋₄ alkyl, C₁₋₄ haloalkyl, CN, OR^{b}, SR^{b}, and NR^{b}R^{b}; wherein each R^{b} is as defined above;
M is selected from O or CR^{h}Rⁱ; wherein R^{h} and Rⁱ are each independently selected from hydrogen, fluoro, and C₁₋₄ alkyl; wherein, said alkyl is optionally substituted by one or more groups selected from halogen, C₁₋₄ haloalkyl, CN, OR^{b}, SR^{b}, NR^{b}R^{b}, C₃₋₆ cycloalkyl, 3-to 8-membered heterocyclyl, aryl, and heteroaryl; wherein each R^{b} are defined as above;
each R⁷ is independently selected from hydrogen, halogen, C₁₋₄ alkyl, C₁₋₄ haloalkyl, C₂₋₄ alkenyl, C₂₋₄ alkynyl, CN, OR^{a}, SR^{a}, NR^{a}R^{a}, C₃₋₆ cycloalkyl, 3-to 8-membered heterocyclyl, aryl, heteroaryl, and heteroaryl C₁₋₄ alkyl; wherein, each R^{a} is defined as above;
R⁸ and R⁹ are each independently selected from the group consisting of hydrogen, fluoro, C₁₋₄ alkyl, C₁₋₄ alkoxy, hydroxy, and C₃₋₆ cycloalkyl; or R⁸ and R⁹ together with the carbon atom to which they are attached form a 3-to 6-membered ring structure which optionally contains 0, 1 or 2 heteroatoms selected from N, O and S;
each R¹⁰ is independently selected from hydrogen, halogen, C₁₋₄ alkyl, C₁₋₄ haloalkyl, C₂₋₄ alkenyl, C₂₋₄ alkynyl, C₃₋₆ cycloalkyl, 3-to 8-membered heterocyclyl, CN, OR^{a}, SR^{a}, and NR^{a}R^{a}; wherein each R^{a} is as defined above;
X is selected from O, S and NRⁿ; wherein Rⁿ is selected from hydrogen and C₁₋₄ alkyl;
each R¹¹ is independently selected from hydrogen, halogen, C₁₋₄ alkyl, C₁₋₄ haloalkyl, C₂₋₄ alkenyl, C₂₋₄ alkynyl, C₃₋₆ cycloalkyl, 3-to 8-membered heterocyclyl, CN, OR^{a}, SR^{a}, NR^{c}R^{c}, and -NR^{d}C(O)R^{g}; wherein, each R^{a} is defined as above; each R^{c} is independently selected from hydrogen, C₁₋₄ alkyl, C₁₋₄ haloalkyl, C₁₋₄ alkoxy-C₁₋₄ alkyl, C₃₋₆ cycloalkyl, 3-to 8-membered heterocyclyl, aryl, and heteroaryl; R^{d} is selected from hydrogen and C₁₋₄ alkyl; R_{g} is defined as above;
m is selected from 0, 1, 2, 3 and 4;
n is selected from 0, 1, 2, 3 and 4;
f is selected from 0, 1, 2 and 3;
g is selected from 0, 1, 2, 3 and 4;
a and b are each independenly selected from 0, 1, 2, 3, 4 and 5; with the proviso that a and b are not 0 simultaneously;
h is selected from 0, 1, 2 and 3;
j is selected from 0, 1, 2 and 3;
k is selected from 0, 1, 2, 3, 4 and 5;
wherein, each of the above-mentioned alkyl, alkenyl, alkynyl, cycloalkyl, heterocyclyl, aryl and heteroaryl is optionally and each independently substituted by 1-3 substituents each independently selected from the group consisting of halogen, C₁₋₄ alkyl, C₁₋₄ haloalkyl, C₂₋₄ alkenyl, C₂₋₄ alkynyl, C₃₋₈ cycloalkyl, 3-to 8-membered heterocyclyl, aryl, heteroaryl, CN, NO₂, OR^{b}, SR^{b}, NR^{d}R^{d}, -C(O)R^{g}, -C(O)OR^{b}, -C(O)NR^{d}R^{d}, -NR^{d}C(O)R^{g}, -NR^{d}S(O)₂R^{g}, and -S(O)₂R^{g}, provided that the chemical structure formed is stable and meaningful; wherein R^{b}, R^{d} and R^{g} are as defined above;
unless otherwise specified, the aryl is aromatic groups having 6 to 12 carbon atoms; the heteroaryl is 5- to 15-membered (preferably 5- to 12-membered) heteroaromatic groups; and the cyclic structure is saturated or unsaturated cyclic groups with or without heteroatoms.

2. The compound of claim 1, wherein the compound (I) is of formula (II): 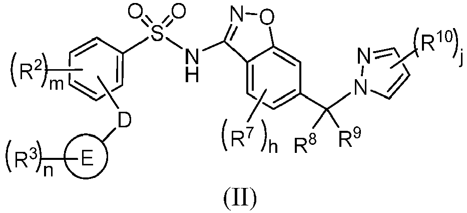 R², R³, R⁷, R⁸, R⁹, R¹⁰, D, E, m, n, h and j are as defined in claim 1.

3. The compound of claim 1, wherein the compound (I) is of formula (III): 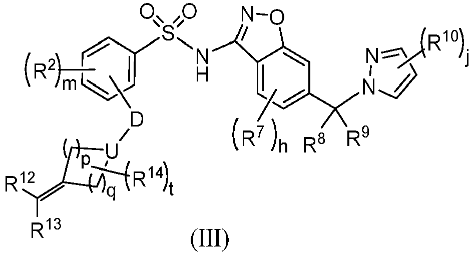
U is selected from N and CR^{k}; wherein R^{k} is selected from hydrogen, halogen and C₁₋₄ alkyl;
each R¹⁴ is independently selected from hydrogen, halogen, C₁₋₄ alkyl, C₁₋₄ haloalkyl, CN, OR^{b}, SR^{b}, and NR^{b}R^{b};
p and q are each independently selected from 0, 1, 2, 3, 4, 5 and 6; with the proviso that p and q are not simultaneously 0;
t is selected from 0, 1, 2, 3 and 4;
R², R⁷, R⁸, R⁹, R¹⁰, R¹², R¹³, R^{b}, D, m, h, and j are as defined in claim 1.

4. The compound of claim 1, wherein the compound (I) is of formula (IV): 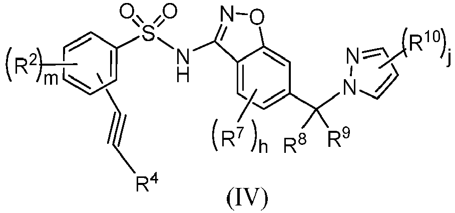 R², R⁴, R⁷, R⁸, R⁹, R¹⁰, m, h and j are as defined in claim 1.

5. The compound of claim 1, wherein the compound (I) is of formula (V): 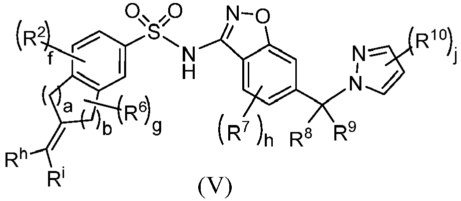 R², R⁶, R⁷, R⁸, R⁹, R¹⁰, R^{h}, Rⁱ, a, b, f, g, h and j are as defined in claim 1.

6. The compound of claim 1, wherein the compound (I) is of formula (VI): 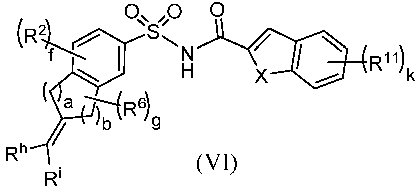 R², R⁶, R¹¹, R^{h}, Rⁱ, X, a, b, f, g and k are as defined in claim 1.

7. The compound of claim 1, wherein the compound (I) is of formula (VII): 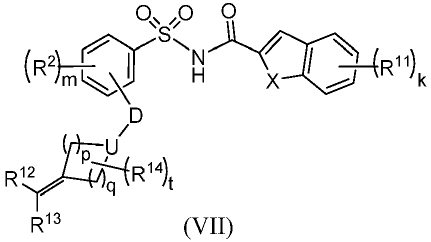
R², R¹¹, R¹², R¹³, D, X, m and k are as defined in claim 1;
U, R¹⁴, p, q and t are as defined in claim 3.

8. The compound of claim 1, wherein the compound (I) is of formula (VIII): 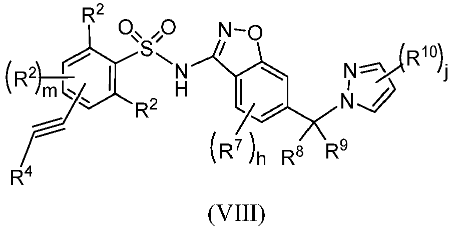
each R² is independently selected from the group consisting of hydrogen, halogen, C₁₋₄ alkyl, C₁₋₄ haloalkyl, C₂₋₄ alkynyl, C₃₋₆ cycloalkyl, CN, OR^{a}, SR^{a}, or NR^{a}R^{a}; wherein each R^{a} is independently selected from hydrogen, C₁₋₄ alkyl, C₁₋₄ haloalkyl, C₃₋₆ cycloalkyl; m is selected from 0, 1 or 2;
R⁴, R⁷, R⁸, R⁹, R¹⁰, h and j are as defined in claim 1.

9. The compound of claim 1, wherein the compound (I) is of formula (IX): 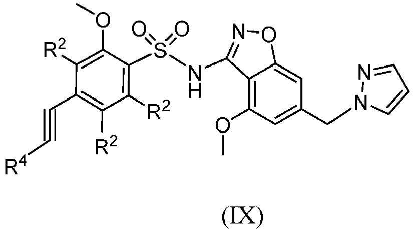
each R² is independently selected from the group consisting of hydrogen, halogen, C₁₋₄ alkyl, C₁₋₄ haloalkyl, C₃₋₆ cycloalkyl, and OR^{a};
R⁴ is selected from the group consisting of hydrogen, C₁₋₄ alkyl, C₃₋₈ cycloalkyl, 3-to 9-membered heterocyclyl, aryl, and heteroaryl; wherein said alkyl, cycloalkyl, heterocyclyl, aryl, or heteroaryl is optionally substituted with one or more groups selected from the group consisting: halogen, CN, OR^{a}, SR^{a}, NR^{a}R^{a}, -C(O)R^{m}, -C(O)OR^{a}, -S(O)₂R^{m}, C₁₋₄ alkyl, C₂₋₄ alkenyl, C₂₋₄ alkynyl, C₃₋₆ cycloalkyl, 3-to 6-membered heterocyclyl, aryl, and heteroaryl; or the cycloalkyl or heterocyclyl is substituted with =T; wherein, T is selected from O or CR¹²R¹³; wherein R¹² and R¹³ are each independently selected from hydrogen, fluorine, and C₁₋₄ alkyl; the alkyl on R¹² or R¹³ is optionally substituted with one or more groups selected from the group consisting of halogen, C₁₋₄ haloalkyl, CN, OR^{b}, SR^{b}, and NR^{b}R^{b};
each R^{a} is independently selected from hydrogen, C₁₋₄ alkyl, C₁₋₄ haloalkyl, and C₃₋₆ cycloalkyl; each R^{b} is independently selected from hydrogen, C₁₋₄ alkyl, and C₁₋₄ haloalkyl; and R^{m} is selected from C₁₋₄ alkyl, C₂₋₄ alkenyl, C₂₋₄ alkynyl and C₃₋₆ cycloalkyl.

10. The compound of claim 1, wherein the compound (I) is of formula (X): 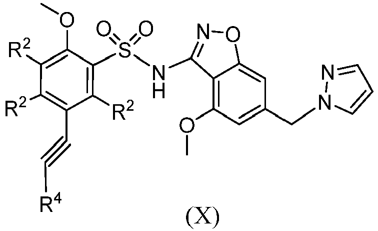
each R² is independently selected from the group consisting of hydrogen, halogen, C₁₋₄ alkyl, C₁₋₄ haloalkyl, C₃₋₆ cycloalkyl, and OR^{a};
R⁴ is selected from the group consisting of hydrogen, C₁₋₄ alkyl, C₃₋₈ cycloalkyl, 3-to 9-membered heterocyclyl, aryl, and heteroaryl; wherein said alkyl, cycloalkyl, heterocyclyl, aryl, or heteroaryl is optionally substituted with one or more groups selected from the group consisting: halogen, CN, OR^{a}, SR^{a}, NR^{a}R^{a}, -C(O)R^{m}, -C(O)OR^{a}, -S(O)₂R^{m}, C₁₋₄ alkyl, C₂₋₄ alkenyl, C₂₋₄ alkynyl, C₃₋₆ cycloalkyl, 3-to 6-membered heterocyclyl, aryl, and heteroaryl; or the cycloalkyl or heterocyclyl is substituted with =T; wherein, T is selected from O and CR¹²R¹³; wherein R¹² and R¹³ are each independently selected from hydrogen, fluorine, or C₁₋₄ alkyl; the alkyl on R¹² or R¹³ is optionally substituted with one or more groups selected from the group consisting of halogen, C₁₋₄ haloalkyl, CN, OR^{b}, SR^{b}, and NR^{b}R^{b};
each R^{a} is independently selected from hydrogen, C₁₋₄ alkyl, C₁₋₄ haloalkyl, and C₃₋₆ cycloalkyl; each R^{b} is independently selected from hydrogen, C₁₋₄ alkyl, and C₁₋₄ haloalkyl; and R^{m} is selected from C₁₋₄ alkyl, C₂₋₄ alkenyl, C₂₋₄ alkynyl and C₃₋₆ cycloalkyl.

11. The compound of claim 1, wherein the compound (I) is of formula (IX): 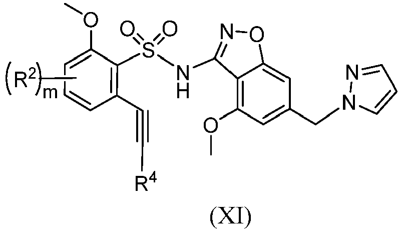
each R² is independently selected from the group consisting of hydrogen, halogen, C₁₋₄ alkyl, C₁₋₄ haloalkyl, C₁₋₄ alkoxy-C₁₋₄ alkyl, C₁₋₄ haloalkoxy-C₁₋₄ alkyl, C₂₋₄ alkenyl, C₂₋₄ haloalkenyl, C₂₋₄ alkynyl, C₃₋₆ cycloalkyl, 3-to 8-membered heterocyclyl, aryl, heteroaryl, CN, OR^{a}, SR^{a}, and NR^{a}R^{a};
R⁴ is selected from the group consisting of hydrogen, C₁₋₄ alkyl, and C₃₋₆ cycloalkyl; wherein the alkyl or cycloalkyl is optionally substituted with one or more groups selected from the group consisting of halogen, CN, OR^{a}, SR^{a} and NR^{a}R^{a};
each R^{a} is independently selected from hydrogen, C₁₋₄ alkyl, C₁₋₄ haloalkyl and C₃₋₆ cycloalkyl;
m is selected from 0, 1, 2 and 3.

12. The compound of claim 1, wherein the compound (I) is of formula (XII): 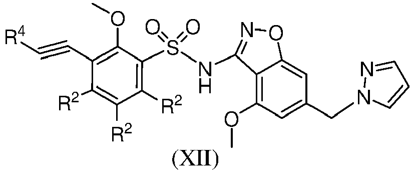 R² and R⁴ are as defined in claim 9.

13. The compound of claim 1, wherein the compound (I) is of formula (VII): 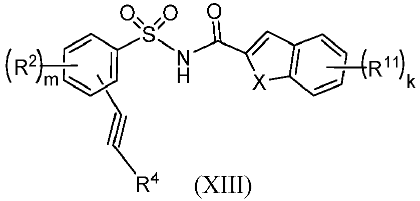 R², R⁴, R¹¹, X, m and k are as defined in claim 1.

14. A compound of the structure shown in formula (XIV), or an optical isomer, a pharmaceutically acceptable salt, a prodrug, a deuterated derivative, a hydrate, or a solvent thereof: 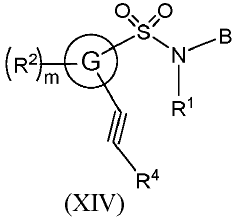
G is selected from C₃₋₈ cycloalkyl, 3-to 10-membered heterocyclyl, aryl (preferably benzene, naphthalene), and heteroaryl (preferably furan, thiophene, pyridine, pyrimidine, pyrazine);
R¹, R², R⁴, B, and m are as defined in claim 1.

15. The compound of claim 1 or 14, wherein the compound of formula (I) or formula (XIV) is selected from the group consisting of 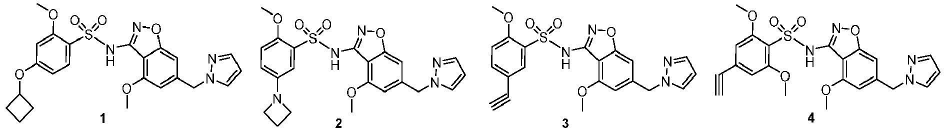 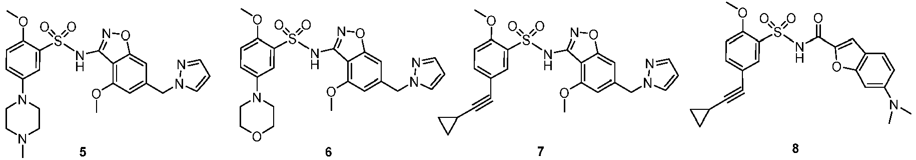 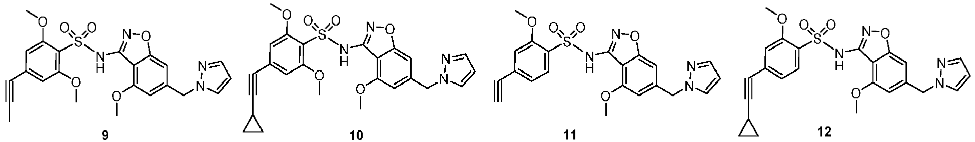 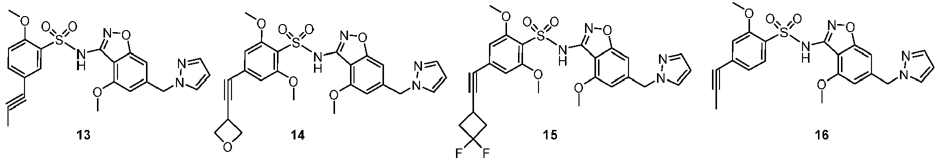 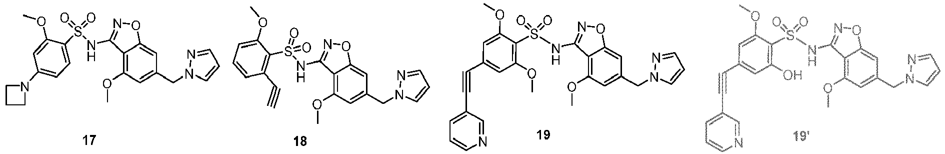 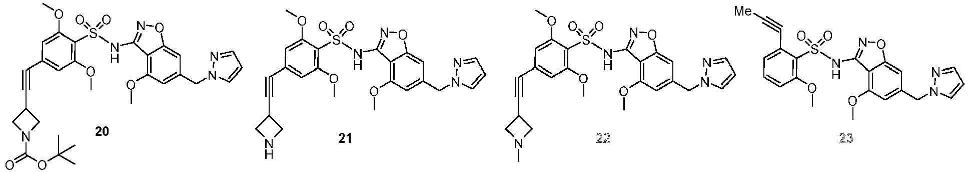 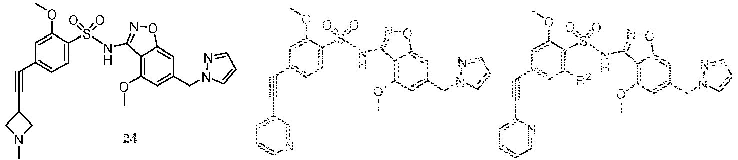 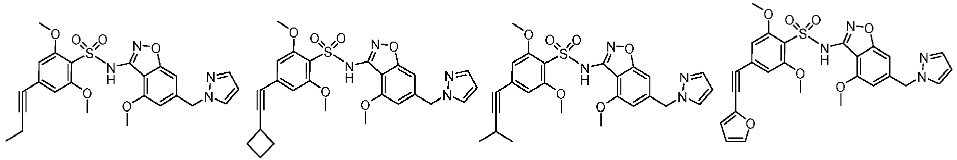 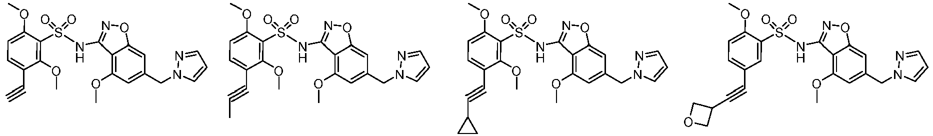 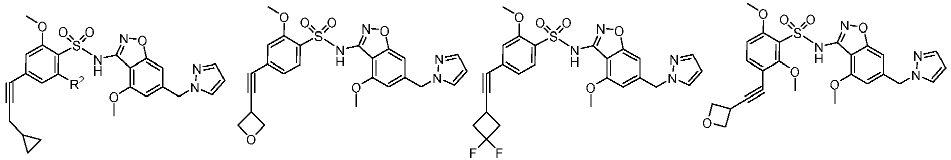 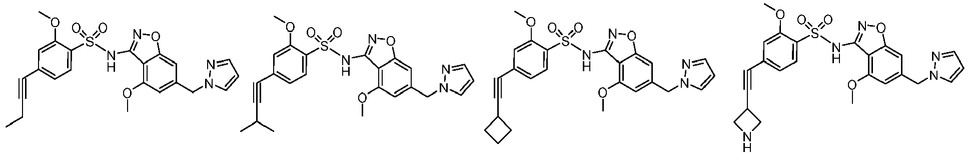 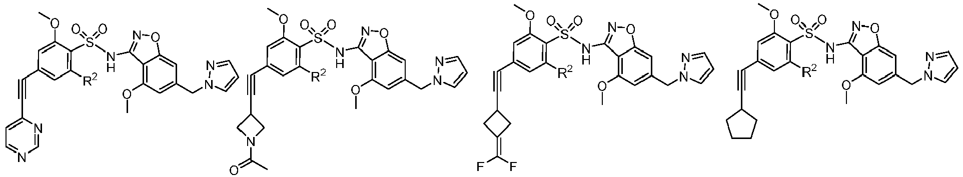 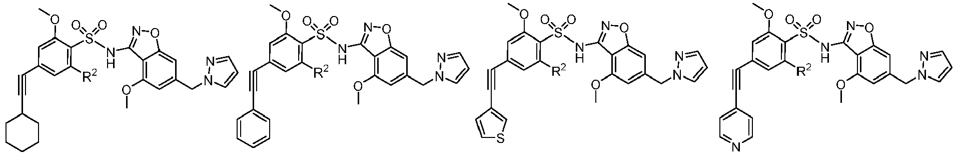 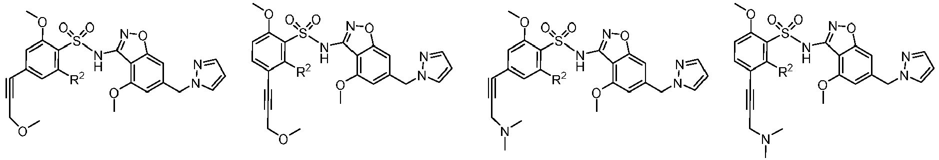 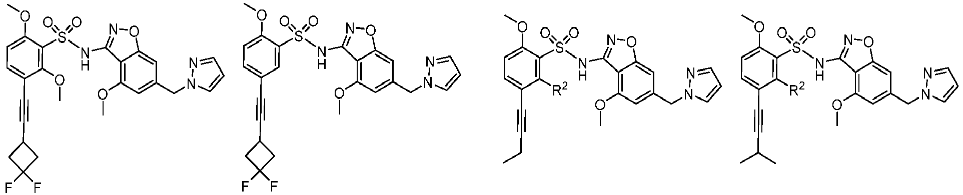 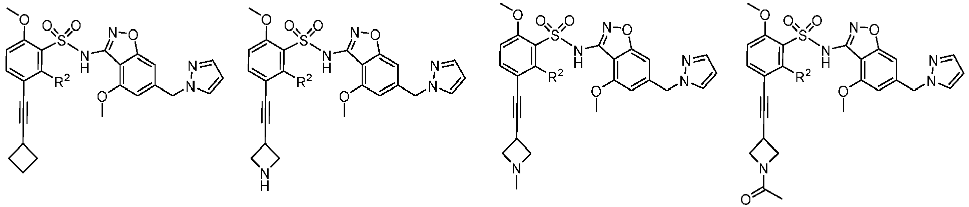 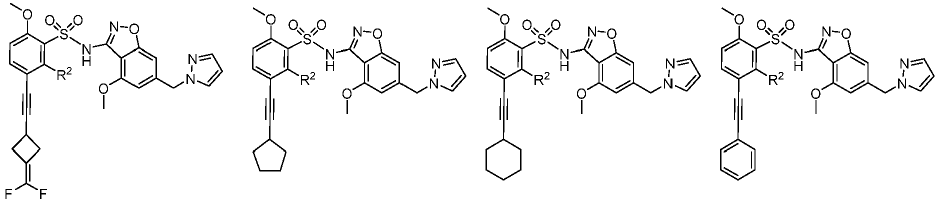 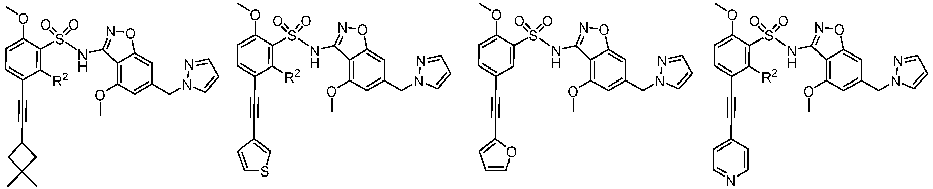 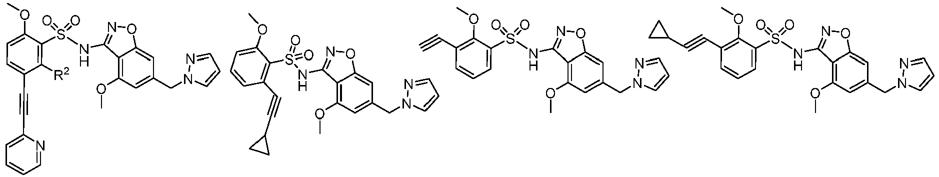 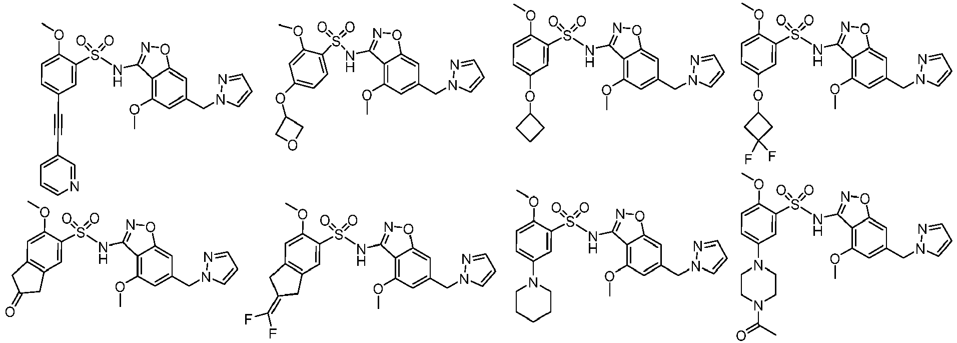 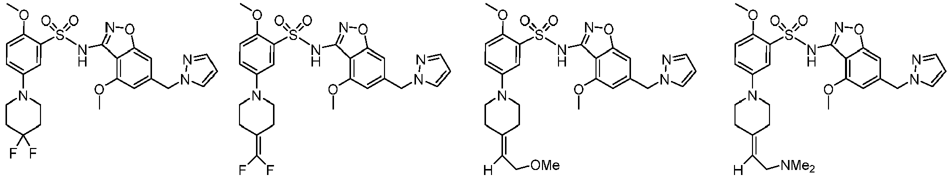 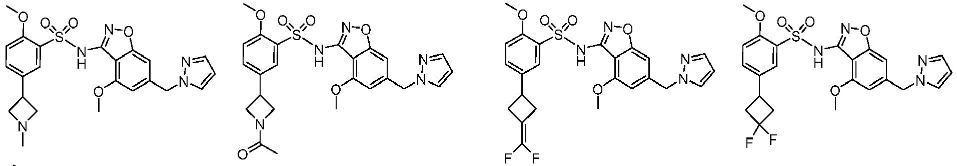 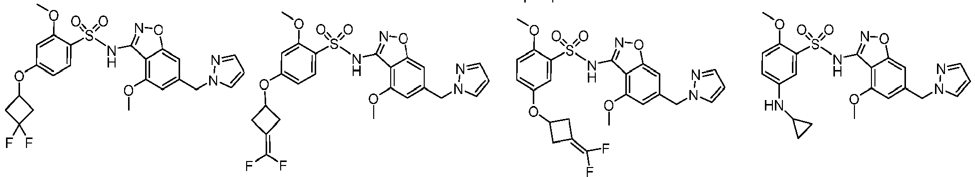 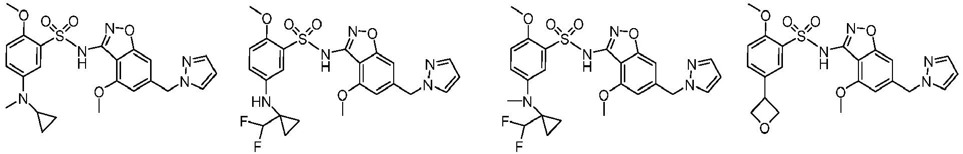 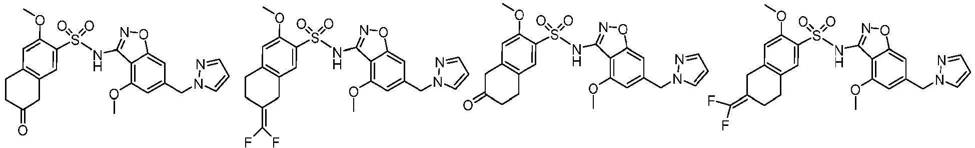 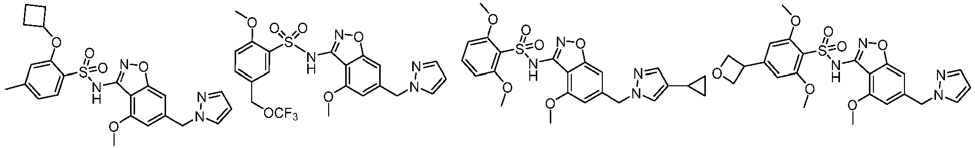 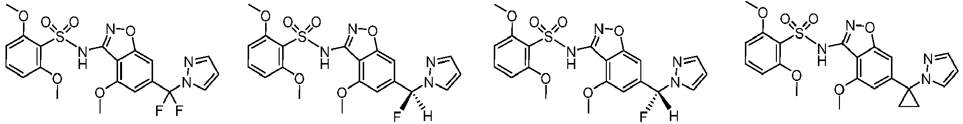 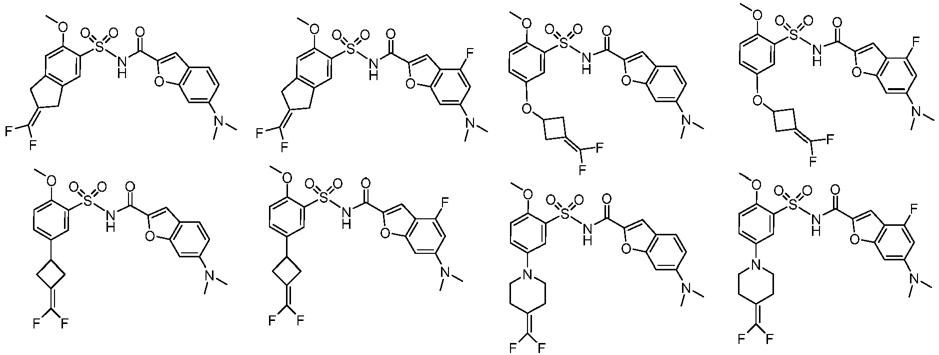 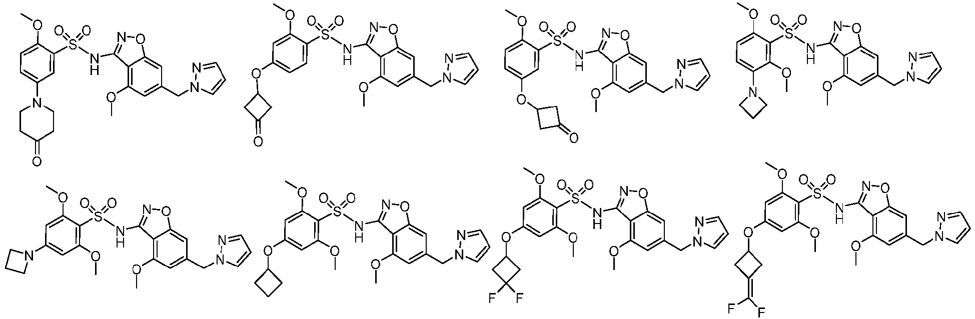 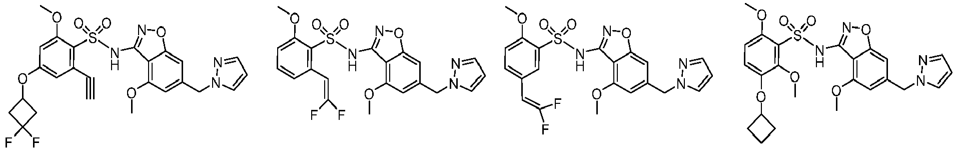 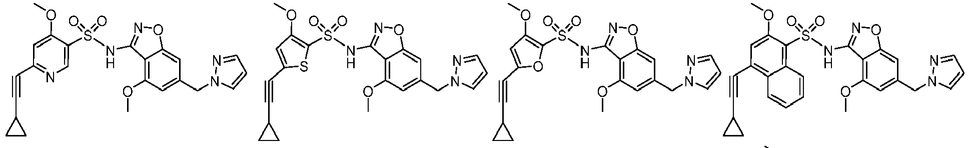 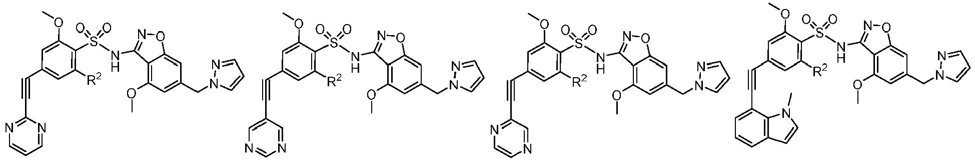 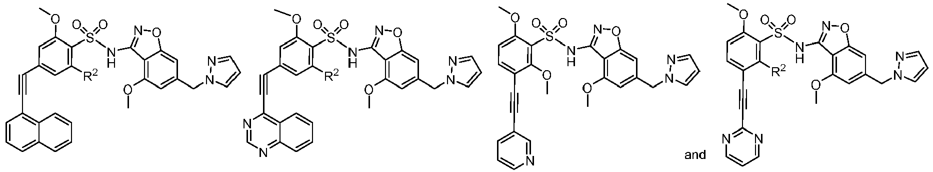 in each of the above compounds, R² is selected from H or OMe.

16. A pharmaceutical composition, wherein comprises the compound of any one of claims 1-15, or the optical, tautomer, or a pharmaceutically acceptable salt, hydrate or solvate thereof, and (2) pharmaceutically acceptable carriers.

17. A use of the compound of any one of claims 1 to 15, or an optical isomer, a pharmaceutically acceptable salt, a prodrug, a deuterated derivative, a hydrate, or a solvent compound thereof in the preparation of a pharmaceutical composition for the treatment of a disease, condition, or condition associated with KAT6 activity or expression.

18. The use of claim 17, **characterized in that** said disease, condition or disease is selected from the group consisting of non-small cell lung cancer, small cell lung cancer, adenocarcinoma of the lung, squamous lung cancer, pancreatic cancer, colon cancer, thyroid cancer, embryonal rhabdomyosarcoma, granulosa cell tumor of the skin, melanoma, hepatocellular carcinoma, intrahepatic cholangiocarcinoma, rectal carcinoma, bladder carcinoma, pharyngolaryngeal cancer, breast carcinoma, vaginal carcinoma, prostate cancer, testicular cancer, brain tumor, glioblastoma, ovarian cancer, head and neck squamous cell carcinoma, cervical cancer, osteosarcoma, esophageal cancer, kidney cancer, skin cancer, gastric cancer, myeloid leukemia, lymphatic leukemia, myeloid fibrosis, B-cell lymphoma, T-cell lymphoma, Hodgkin's lymphoma, non-Hodgkin's lymphoma, monocytic leukemia, polycythemia megalosplenica, Eosinophilic leukocytosis syndrome multiple, bone marrow cancer, and various other solid and hematologic tumors, etc..
